# EUROPEAN PATENT APPLICATION

(11) **EP 4 656 724 A2**
(43) Date of publication of application: **03.12.2025**
(21) Application number: 25209340.6
(22) Date of filing: 18.02.2022
(51) Int. Cl.: C12N 15/69

(54) **TARGET PROTEIN PRODUCTION METHOD**

(30) Priority: 19.02.2021 JP 2021025664
(62) Divisional of application: 24210049.3
(71) Applicant: Nagase Diagnostics Co., Ltd., Tokyo 103-0024 (JP); National Institute of Advanced Industrial Science and Technology, Chiyoda-ku Tokyo 100-8921 (JP)
(72) Inventor: YOSHIDA, Keitaro, Sapporo-shi (JP); YASUTAKE, Yoshiaki, Sapporo-shi (JP); TAMURA, Tomohiro, Sapporo-shi (JP); KONISHI, Kenji, Tokyo (JP); SAKASEGAWA, Shin-ichi, Tokyo (JP); MURAMATSU, Shuji, Tokyo (JP)
(74) Representative: Forstmeyer, Dietmar

(57) **Abstract**

A method for producing a protein encoded by a target gene, comprising the step of
expressing the target gene in a bacterium of the genus *Burkholderia,* wherein the bacterium lacks one or more genes selected from the group consisting of BSFP_068740, BSFP_068730, and BSFP_068720, or has inhibited expression of the genes or proteins encoded by the genes.

## Description

### Technical Field

The present invention relates to a method for producing a target protein in a bacterium of the genus *Burkholderia,* etc.

### Background Art

Bacteria of the genus *Burkholderia* are gram negative bacteria that secrete and produce useful lipase (Patent Literature 1) and can perform the expression and secretion of extracellularly secretory lipase or the like, which is difficult to express in an active form in *E. coli.* Therefore, utilization thereof as a platform for useful protein expression systems has been reported (see Patent Literatures 2 and 3).

However, the amount of a recombinant protein produced using a bacterium of the genus *Burkholderia* is smaller than that in a host-vector system using E. *coli,* yeast/filamentous bacterium, or the like. Therefore, most cases do not utilize this approach, whereas some cases employ a host-vector system using E. *coli,* not a bacterium of the genus *Burkholderia,* in order to produce lipase derived from the genus *Burkholderia* (Patent Literature 4 and Non Patent Literature 1).

### Citation List

### Patent Literature

Patent Literature 1: Japanese Patent Laid-Open No. 11-253157
Patent Literature 2: Japanese Translation of PCT International Application Publication No. 2008-543294
Patent Literature 3: Japanese Patent Laid-Open No. 2019-205402
Patent Literature 4: Japanese Patent No. 5993229

### Non Patent Literature

Non Patent Literature 1: M El Khattabi et al., J. Bio Chem., 275 (35), 26885-91 (2000)

### Summary of Invention

### Technical Problem

An object of the present invention is to provide a novel approach that enables a recombinant protein to be expressed in a large amount in a bacterium of the genus *Burkholderia* which is not utilized as a platform for useful protein expression systems in most cases.

### Solution to Problem

The present inventors have found that a gene on a particular chromosome in a bacterium of the genus *Burkholderia* is functionally inhibited, whereby an expression vector is stable in the bacterium of the genus *Burkholderia* while more preferred properties for stable protein production are imparted to the bacterium. A novel promoter capable of increasing the expression level of a target protein has also been newly found.

Specifically, the present application encompasses the following invention.
[1] A method for producing a protein encoded by a target gene, comprising the step of
   expressing the target gene in a bacterium of the genus *Burkholderia,* wherein the bacterium lacks one or more genes selected from the group consisting of BSFP_068740, BSFP_068730, and BSFP_068720, or has inhibited expression of the genes or proteins encoded by the genes.
[2] The method according to [1], wherein
   the gene BSFP_068740 comprises any one of the following DNAs (i) to (viii):
   (i) DNA consisting of the nucleotide sequence of SEQ ID NO: 1;
   (ii) DNA capable of hybridizing under stringent conditions to DNA consisting of a nucleotide sequence complementary to the nucleotide sequence of SEQ ID NO: 1, wherein a protein encoded thereby has a function of decreasing a copy number of an expression construct in the bacterium of the genus *Burkholderia,* or reducing stability thereof;
   (iii) DNA consisting of a nucleotide sequence derived from the nucleotide sequence of SEQ ID NO: 1 by the deletion, substitution, or addition of one or a few bases, wherein a protein encoded thereby has a function of decreasing a copy number of an expression construct in the bacterium of the genus *Burkholderia,* or reducing stability thereof;
   (iv) DNA consisting of a nucleotide sequence having at least 80% or higher sequence identity to SEQ ID NO: 1, wherein a protein encoded thereby has a function of decreasing a copy number of an expression construct in the bacterium of the genus *Burkholderia,* or reducing stability thereof;
   (v) DNA consisting of a nucleotide sequence encoding a protein consisting of the amino acid sequence of SEQ ID NO: 2;
   (vi) DNA capable of hybridizing under stringent conditions to DNA consisting of a nucleotide sequence encoding a protein consisting of the amino acid sequence of SEQ ID NO: 2, wherein a protein encoded thereby has a function of decreasing a copy number of an expression construct in the bacterium of the genus *Burkholderia,* or reducing stability thereof;
   (vii) DNA consisting of a nucleotide sequence encoding a protein consisting of an amino acid sequence derived from the amino acid sequence of SEQ ID NO: 2 by the deletion, substitution, or addition of one or a few amino acids, wherein the protein has a function of decreasing a copy number of an expression construct in the bacterium of the genus *Burkholderia,* or reducing stability thereof; and
   (viii) DNA consisting of a nucleotide sequence encoding a protein consisting of an amino acid sequence having at least 80% or higher sequence identity to SEQ ID NO: 2, wherein the protein has a function of decreasing a copy number of an expression construct in the bacterium of the genus *Burkholderia,* or reducing stability thereof.
[3] The method according to [1] or [2], wherein
   the gene BSFP_068730 comprises any one of the following DNAs (i) to (viii):
   (i) DNA consisting of the nucleotide sequence of SEQ ID NO: 3;
   (ii) DNA capable of hybridizing under stringent conditions to DNA consisting of a nucleotide sequence complementary to the nucleotide sequence of SEQ ID NO: 3, wherein a protein encoded thereby has a function of decreasing a copy number of an expression construct in the bacterium of the genus *Burkholderia,* or reducing stability thereof;
   (iii) DNA consisting of a nucleotide sequence derived from the nucleotide sequence of SEQ ID NO: 3 by the deletion, substitution, or addition of one or a few bases, wherein a protein encoded thereby has a function of decreasing a copy number of an expression construct in the bacterium of the genus *Burkholderia,* or reducing stability thereof;
   (iv) DNA consisting of a nucleotide sequence having at least 80% or higher sequence identity to SEQ ID NO: 3, wherein a protein encoded thereby has a function of decreasing a copy number of an expression construct in the bacterium of the genus *Burkholderia,* or reducing stability thereof;
   (v) DNA consisting of a nucleotide sequence encoding a protein consisting of the amino acid sequence of SEQ ID NO: 4;
   (vi) DNA capable of hybridizing under stringent conditions to DNA consisting of a nucleotide sequence encoding a protein consisting of the amino acid sequence of SEQ ID NO: 4, wherein a protein encoded thereby has a function of decreasing a copy number of an expression construct in the bacterium of the genus *Burkholderia,* or reducing stability thereof;
   (vii) DNA consisting of a nucleotide sequence encoding a protein consisting of an amino acid sequence derived from the amino acid sequence of SEQ ID NO: 4 by the deletion, substitution, or addition of one or a few amino acids, wherein the protein has a function of decreasing a copy number of an expression construct in the bacterium of the genus *Burkholderia,* or reducing stability thereof; and
   (viii) DNA consisting of a nucleotide sequence encoding a protein consisting of an amino acid sequence having at least 80% or higher sequence identity to SEQ ID NO: 4, wherein the protein has a function of decreasing a copy number of an expression construct in the bacterium of the genus *Burkholderia,* or reducing stability thereof.
[4] The method according to any of [1] to [3], wherein
   the gene BSFP_068720 comprises any one of the following DNAs (i) to (viii):
   (i) DNA consisting of the nucleotide sequence of SEQ ID NO: 5;
   (ii) DNA capable of hybridizing under stringent conditions to DNA consisting of a nucleotide sequence complementary to the nucleotide sequence of SEQ ID NO: 5, wherein a protein encoded thereby has a function of decreasing a copy number of an expression construct in the bacterium of the genus *Burkholderia,* or reducing stability thereof;
   (iii) DNA consisting of a nucleotide sequence derived from the nucleotide sequence of SEQ ID NO: 5 by the deletion, substitution, or addition of one or a few bases, wherein a protein encoded thereby has a function of decreasing a copy number of an expression construct in the bacterium of the genus *Burkholderia,* or reducing stability thereof;
   (iv) DNA consisting of a nucleotide sequence having at least 80% or higher sequence identity to SEQ ID NO: 5, wherein a protein encoded thereby has a function of decreasing a copy number of an expression construct in the bacterium of the genus *Burkholderia,* or reducing stability thereof;
   (v) DNA consisting of a nucleotide sequence encoding a protein consisting of the amino acid sequence of SEQ ID NO: 6;
   (vi) DNA capable of hybridizing under stringent conditions to DNA consisting of a nucleotide sequence encoding a protein consisting of the amino acid sequence of SEQ ID NO: 6, wherein a protein encoded thereby has a function of decreasing a copy number of an expression construct in the bacterium of the genus *Burkholderia,* or reducing stability thereof;
   (vii) DNA consisting of a nucleotide sequence encoding a protein consisting of an amino acid sequence derived from the amino acid sequence of SEQ ID NO: 6 by the deletion, substitution, or addition of one or a few amino acids, wherein the protein has a function of decreasing a copy number of an expression construct in the bacterium of the genus *Burkholderia,* or reducing stability thereof; and
   (viii) DNA consisting of a nucleotide sequence encoding a protein consisting of an amino acid sequence having at least 80% or higher sequence identity to SEQ ID NO: 6, wherein the protein has a function of decreasing a copy number of an expression construct in the bacterium of the genus *Burkholderia,* or reducing stability thereof.
[5] The method according to any of [1] to [4], wherein the target gene is expressibly linked to the expression construct.
[6] The method according to any of [2] to [5], wherein the expression construct is an expression vector.
[7] The method according to [6], wherein the expression construct is a plasmid capable of replicating in the bacterium of the genus *Burkholderia.*
[8] The method according to [7], wherein the plasmid is a broad-host-range vector.
[9] The method according to [8], wherein the broad-host-range vector is pBBR122, pSa, or RK2.
[10] The method according to any of [2] to [9], wherein the expression construct comprises a promoter that controls the expression of the target gene.
[11] The method according to [10], wherein the promoter is derived from the bacterium of the genus *Burkholderia.*
[12] The method according to any of [1] to [11], wherein
   the promoter comprises any one of the following DNAs (i) to (iv):
   (i) DNA that consists of a sequence of more than 80 consecutive nucleotides in the nucleotide sequence of SEQ ID NO: 7, and has promoter activity;
   (ii) DNA that is capable of hybridizing under stringent conditions to DNA consisting of a complementary sequence of more than 80 consecutive nucleotides in the nucleotide sequence of SEQ ID NO: 7, and has promoter activity;
   (iii) DNA that consists of a nucleotide sequence derived from a sequence of more than 80 consecutive nucleotides in the nucleotide sequence of SEQ ID NO: 7 by the deletion, substitution, or addition of one or a few bases, and has promoter activity; and
   (iv) DNA that consists of a nucleotide sequence having at least 80% or higher sequence identity to a sequence of more than 80 consecutive nucleotides in SEQ ID NO: 7, and has promoter activity.
[13] The method according to [12], wherein
   the promoter comprises any one of the following DNAs (i) to (iv):
   (i) DNA consisting of the nucleotide sequence of SEQ ID NO: 8;
   (ii) DNA that is capable of hybridizing under stringent conditions to DNA consisting of a nucleotide sequence complementary to the nucleotide sequence of SEQ ID NO: 8, and has promoter activity;
   (iii) DNA that consists of a nucleotide sequence derived from the nucleotide sequence of SEQ ID NO: 8 by the deletion, substitution, or addition of one or a few bases, and has promoter activity; and
   (iv) DNA that consists of a nucleotide sequence having at least 80% or higher sequence identity to SEQ ID NO: 8, and has promoter activity.
[14] The method according to any of [1] to [13], wherein
   the promoter comprises any one of the following DNAs (i) to (iv):
   (i) DNA that consists of a sequence of more than 100 consecutive nucleotides in the nucleotide sequence of SEQ ID NO: 9, and has promoter activity;
   (ii) DNA that is capable of hybridizing under stringent conditions to DNA consisting of a complementary sequence of more than 100 consecutive nucleotides in the nucleotide sequence of SEQ ID NO: 9, and has promoter activity;
   (iii) DNA that consists of a nucleotide sequence derived from a sequence of more than 100 consecutive nucleotides in the nucleotide sequence of SEQ ID NO: 9 by the deletion, substitution, or addition of one or a few bases, and has promoter activity; and
   (iv) DNA that consists of a nucleotide sequence having at least 80% or higher sequence identity to a sequence of more than 100 consecutive nucleotides in SEQ ID NO: 9, and has promoter activity.
[15] The method according to [14], wherein
   the promoter comprises any one of the following DNAs (i) to (iv):
   (i) DNA consisting of the nucleotide sequence of SEQ ID NO: 10;
   (ii) DNA that is capable of hybridizing under stringent conditions to DNA consisting of a nucleotide sequence complementary to the nucleotide sequence of SEQ ID NO: 10, and has promoter activity;
   (iii) DNA that consists of a nucleotide sequence derived from the nucleotide sequence of SEQ ID NO: 10 by the deletion, substitution, or addition of one or a few bases, and has promoter activity; and
   (iv) DNA that consists of a nucleotide sequence having at least 80% or higher sequence identity to SEQ ID NO: 10, and has promoter activity.
[16] The method according to any of [1] to [15], wherein the target gene encodes esterase.
[17] The method according to [16], wherein
   the gene encoding esterase comprises any one of the following DNAs (i) to (iv):
   (i) DNA consisting of a nucleotide sequence of any one of SEQ ID NOs: 11 to 13;
   (ii) DNA capable of hybridizing under stringent conditions to DNA consisting of a nucleotide sequence complementary to a nucleotide sequence of any one of SEQ ID NOs: 11 to 13, wherein a protein encoded thereby has esterase activity;
   (iii) DNA consisting of a nucleotide sequence derived from a nucleotide sequence of any one of SEQ ID NOs: 11 to 13 by the deletion, substitution, or addition of one or a few bases, wherein a protein encoded thereby has esterase activity; and
   (iv) DNA consisting of a nucleotide sequence having at least 80% or higher sequence identity to a nucleotide sequence of any one of SEQ ID NOs: 11 to 13, wherein a protein encoded thereby has esterase activity.
[18] The method according to any of [1] to [17], wherein the expression construct comprises DNA encoding foldase.
[19] The method according to [18], wherein
   the DNA encoding foldase comprises any one of the following DNAs (i) to (iv):
   (i) DNA consisting of a nucleotide sequence of any one of SEQ ID NOs: 14 to 16;
   (ii) DNA capable of hybridizing under stringent conditions to DNA consisting of a nucleotide sequence complementary to a nucleotide sequence of any one of SEQ ID NOs: 14 to 16, wherein a protein encoded thereby has foldase activity;
   (iii) DNA consisting of a nucleotide sequence derived from a nucleotide sequence of any one of SEQ ID NOs: 14 to 16 by the deletion, substitution, or addition of one or a few bases, wherein a protein encoded thereby has foldase activity; and
   (iv) DNA consisting of a nucleotide sequence having at least 80% or higher sequence identity to a nucleotide sequence of any one of SEQ ID NOs: 14 to 16, wherein a protein encoded thereby has foldase activity.
[20] The method according to any of [1] to [19], wherein the target gene further encodes a signal sequence.
[21] The method according to any of [1] to [20], wherein the bacterium of the genus *Burkholderia* is *Burkholderia stabilis.*
[22] A promoter consisting of any one of the following DNAs (i) to (iv):
   (i) DNA that consists of a sequence of more than 80 consecutive nucleotides in the nucleotide sequence of SEQ ID NO: 7, and has promoter activity;
   (ii) DNA that is capable of hybridizing under stringent conditions to DNA consisting of a complementary sequence of more than 80 consecutive nucleotides in the nucleotide sequence of SEQ ID NO: 7, and has promoter activity;
   (iii) DNA that consists of a nucleotide sequence derived from a sequence of more than 80 consecutive nucleotides in the nucleotide sequence of SEQ ID NO: 7 by the deletion, substitution, or addition of one or a few bases, and has promoter activity; and
   (iv) DNA that consists of a nucleotide sequence having at least 80% or higher sequence identity to a sequence of more than 80 consecutive nucleotides in SEQ ID NO: 7, and has promoter activity.
[23] The promoter according to [22], wherein
   the promoter comprises any one of the following DNAs (i) to (iv):
   (i) DNA consisting of the nucleotide sequence of SEQ ID NO: 8;
   (ii) DNA that is capable of hybridizing under stringent conditions to DNA consisting of a nucleotide sequence complementary to the nucleotide sequence of SEQ ID NO: 8, and has promoter activity;
   (iii) DNA that consists of a nucleotide sequence derived from the nucleotide sequence of SEQ ID NO: 8 by the deletion, substitution, or addition of one or a few bases, and has promoter activity; and
   (iv) DNA that consists of a nucleotide sequence having at least 80% or higher sequence identity to SEQ ID NO: 8, and has promoter activity.
[24] A promoter consisting of any one of the following DNAs (i) to (iv):
   (i) DNA that consists of a sequence of more than 100 consecutive nucleotides in the nucleotide sequence of SEQ ID NO: 9, and has promoter activity;
   (ii) DNA that is capable of hybridizing under stringent conditions to DNA consisting of a complementary sequence of more than 100 consecutive nucleotides in the nucleotide sequence of SEQ ID NO: 9, and has promoter activity;
   (iii) DNA that consists of a nucleotide sequence derived from a sequence of more than 100 consecutive nucleotides in the nucleotide sequence of SEQ ID NO: 9 by the deletion, substitution, or addition of one or a few bases, and has promoter activity; and
   (iv) DNA that consists of a nucleotide sequence having at least 80% or higher sequence identity to a sequence of more than 100 consecutive nucleotides in SEQ ID NO: 9, and has promoter activity.
[25] The promoter according to [24], wherein
   the promoter comprises any one of the following DNAs (i) to (iv):
   (i) DNA consisting of the nucleotide sequence of SEQ ID NO: 10;
   (ii) DNA that is capable of hybridizing under stringent conditions to DNA consisting of a nucleotide sequence complementary to the nucleotide sequence of SEQ ID NO: 10, and has promoter activity;
   (iii) DNA that consists of a nucleotide sequence derived from the nucleotide sequence of SEQ ID NO: 10 by the deletion, substitution, or addition of one or a few bases, and has promoter activity; and
   (iv) DNA that consists of a nucleotide sequence having at least 80% or higher sequence identity to SEQ ID NO: 10, and has promoter activity.
[26] A mutant strain of a bacterium of the genus *Burkholderia* engineered to have inhibited expression of one or more genes selected from the group consisting of BSFP_068740, BSFP_068730, and BSFP_068720 or proteins encoded by the genes.
[27] The mutant strain according to [26], wherein the mutant strain comprises an expression construct, and the expression construct comprises a promoter that controls the expression of the target gene.
[28] The mutant strain according to [27], wherein the promoter is a promoter according to any of [22] to [25].
[29] A method for maintaining an expression construct in a bacterium of the genus *Burkholderia,* comprising the step of
   culturing a bacterium of the genus *Burkholderia,* wherein the bacterium of the genus *Burkholderia* lacks one or more genes selected from the group consisting of BSFP_068740, BSFP_068730, and BSFP_068720, or has inhibited expression of the genes or proteins encoded by the genes, and the bacterium comprises the expression construct.
[30] The method according to [29], wherein the cultured bacterium of the genus *Burkholderia* has an increased copy number of the expression construct or increased stability thereof.

### Advantageous Effects of Invention

The present invention enables a target protein to be highly produced as compared with a bacterium of the genus *Burkholderia* whose gene on a particular chromosome is not functionally inhibited, and is also capable of increasing the stability of an expression vector in a bacterium of the genus *Burkholderia.*

### Brief Description of Drawings

[Figure 1] Figure 1 shows a process of preparing a vector for gene disruption.
[Figure 2] Figure 2 shows electrophoresis results of PCR products of a wild-type strain (1) and a disrupted gene moiety of a gene disruptant (2) (lower diagram), and a schematic view of the gene of the wild-type strain and the disrupted gene (upper diagram).

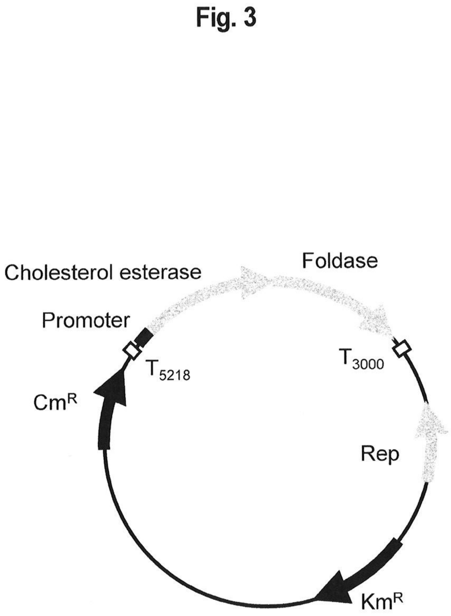

[Figure 3] Figure 3 shows a cholesterol esterase expression vector.
[Figure 4] Figure 4 shows a copy number per bacterium of pBBR122 extracted from a wild-type strain, a BSFP_068720 disruptant, a BSFP_068730 disruptant, a BSFP_068740 disruptant, or a chromosome 3 deletion mutant transfected with pBBR122.
[Figure 5] Figure 5 shows electrophoresis results of pBBR122 extracted from a wild-type strain, a BSFP_068720 disruptant, a BSFP_068730 disruptant, a BSFP_068740 disruptant, or a chromosome 3 deletion mutant transfected with pBBR122.
[Figure 6] Figure 6 shows cholesterol esterase activity in a wild-type strain, a BSFP_068720 disruptant, a BSFP_068730 disruptant, a BSFP_068740 disruptant, or a chromosome 3 deletion mutant transfected with pBBR122 containing no cholesterol esterase gene or pBBR122-182 containing cholesterol esterase gene.
[Figure 7] Figure 7 shows electrophoresis results of pSa extracted from a wild-type strain, a BSFP_068720 disruptant, a BSFP_068730 disruptant, a BSFP_068740 disruptant, or a chromosome 3 deletion mutant transfected with pSa.
[Figure 8] Figure 8 shows cholesterol esterase activity in a wild-type strain, a BSFP_068720 disruptant, a BSFP_068730 disruptant, a BSFP_068740 disruptant, or a chromosome 3 deletion mutant transfected with pRK2-182.
[Figure 9] Figure 9 shows lipase activity in a wild-type strain, a BSFP_068720 disruptant, a BSFP_068730 disruptant, a BSFP_068740 disruptant, or a chromosome 3 deletion mutant transfected with a vector containing lipase gene derived from *Burkholderia cepacia.*
[Figure 10] Figure 10 shows lipase activity in a wild-type strain, a BSFP_068720 disruptant, a BSFP_068730 disruptant, a BSFP_068740 disruptant, or a chromosome 3 deletion mutant transfected with a vector containing lipase gene derived from *Burkholderia plantarii.*
[Figure 11] Figure 11 shows an expression level of cholesterol esterase when 14020 promoter and 48230 promoter were truncated.
[Figure 12] Figure 12 shows electrophoresis results of amplification products obtained by PCR using *Burkholderia silvatlantica* total DNA and *Burkholderia stabilis* total DNA as templates and primers of SEQ ID NOs: 98 and 99.

### Description of Embodiments

The first embodiment provides a method for producing a protein encoded by a target gene, comprising the step of expressing the target gene in a bacterium of the genus *Burkholderia,* wherein the bacterium lacks one or more genes selected from the group consisting of genes, for example, BSFP_068740, BSFP_068730, and BSFP_068720, on a particular chromosome in the bacterium of the genus *Burkholderia,* for example, chromosome 3 for *Burkholderia stabilis,* or has inhibited expression of the genes or proteins encoded by the genes.

A nucleotide sequence encoding each of the proteins is not particularly limited and may be, for example, a nucleotide sequence changed from the nucleotide sequence of SEQ ID NO: 1, 3 or 5 according to the codon usage of a host such as a bacterium of the genus *Burkholderia* (e.g., *Burkholderia stabilis*)*.* As long as the proteins are proteins having a function of decreasing a copy number of an expression construct or reducing stability thereof, the nucleotide sequence may be a nucleotide sequence encoding an amino acid sequence substantially equivalent to each protein, or may be, for example, a nucleotide sequence encoding an equivalent amino acid sequence derived from the amino acid sequence of each protein by the mutation of some amino acids, for example, by the deletion, substitution, or addition of one or more amino acids, which are not involved in the function. It is particularly preferred that the nucleotide sequence of SEQ ID NO: 1, 3 or 5 should be wholly or partially included.

Bacteria of the genus *Burkholderia* which are gram negative aerobic rods are gram negative aerobic rods that may be used as a platform for useful protein expression systems, and 60 or more species including *Burkholderia stabilis* have been registered. Among the bacteria of the genus *Burkholderia,* for example, *Burkholderia stabilis* FERMP-21014 (Genome Announcements Volume 5 Issue 29 e00636-17) has three chromosomes, chromosome 1 (3.6 Mbp), chromosome 2 (3.2 Mbp), and chromosome 3 (0.9 Mbp), and 849 genes reside on the smallest chromosome 3. The deletion of a particular chromosome in a bacterium of the genus *Burkholderia* can increase the amount of a target protein produced as compared with this bacterium without such deletion. Although not wishing to be bound by any theory, a gene having a function of eliminating the stability of an expression vector such as a plasmid, residing on the particular chromosome is functionally inhibited, whereby the stability of the expression vector is increased, resulting in an increased amount of a protein produced.

As used herein, the "bacterium of the genus *Burkholderia"* means any bacterium of the genus *Burkholderia.* As long as the gene having a function of eliminating the stability of an expression vector such as a plasmid is functionally inhibited, the bacterium of the genus *Burkholderia* may occur naturally or may be artificially created. The bacterium of the genus *Burkholderia* is preferably *Burkholderia stabilis* or *Burkholderia silvatlantica.*

The genomic sequence of *Burkholderia stabilis* FERMP-21014 is known in the art (https://www.ncbi.nlm.nih.gov/genome/45559?genome_assembl y_id=331877), and a gene sequence constituting, for example, chromosome 3, is also known in the art (https://www.ncbi.nlm.nih.gov/nuccore/AP018113.1). Examples of the gene having a function of eliminating the stability of an expression vector such as a plasmid include BSFP_068740, BSFP_068730, and BSFP_068720 of *Burkholderia stabilis* FERMP-21014. All of BSFP_068740, BSFP_068730, and BSFP_068720 residing on chromosome 3 may have different functions or may have the same function. The inhibition of one or more functions of each gene can improve the stability of an expression vector. BSFP No. represents a locus tag provided by NCBI (National Center for Biotechnology Information).

In a preferred aspect, the gene BSFP_068740 may comprise any one of the following DNAs (i) to (viii):
(i) DNA consisting of the nucleotide sequence of SEQ ID NO: 1;
(ii) DNA capable of hybridizing under stringent conditions to DNA consisting of a nucleotide sequence complementary to the nucleotide sequence of SEQ ID NO: 1, wherein a protein encoded thereby has a function of decreasing a copy number of an expression construct in the bacterium of the genus *Burkholderia,* or reducing stability thereof;
(iii) DNA consisting of a nucleotide sequence derived from the nucleotide sequence of SEQ ID NO: 1 by the deletion, substitution, or addition of one or more, preferably one or a few bases, wherein a protein encoded thereby has a function of decreasing a copy number of an expression construct in the bacterium of the genus *Burkholderia,* or reducing stability thereof;
(iv) DNA consisting of a nucleotide sequence having at least 80% or higher, preferably 90% or higher sequence homology, preferably sequence identity to SEQ ID NO: 1, wherein a protein encoded thereby has a function of decreasing a copy number of an expression construct in the bacterium of the genus *Burkholderia,* or reducing stability thereof;
(v) DNA consisting of a nucleotide sequence encoding a protein consisting of the amino acid sequence of SEQ ID NO: 2;
(vi) DNA capable of hybridizing under stringent conditions to DNA consisting of a nucleotide sequence encoding a protein consisting of the amino acid sequence of SEQ ID NO: 2, wherein a protein encoded thereby has a function of decreasing a copy number of an expression construct in the bacterium of the genus *Burkholderia,* or reducing stability thereof;
(vii) DNA consisting of a nucleotide sequence encoding a protein consisting of an amino acid sequence derived from the amino acid sequence of SEQ ID NO: 2 by the deletion, substitution, or addition of one or a few amino acids, wherein the protein has a function of decreasing a copy number of an expression construct in the bacterium of the genus *Burkholderia,* or reducing stability thereof; and
(viii) DNA consisting of a nucleotide sequence encoding a protein consisting of an amino acid sequence having at least 80% or higher, preferably 90% or higher sequence homology, preferably sequence identity to SEQ ID NO: 2, wherein the protein has a function of decreasing a copy number of an expression construct in the bacterium of the genus *Burkholderia,* or reducing stability thereof.

In a preferred aspect, the gene BSFP_068730 may comprise any one of the following DNAs (i) to (viii):
(i) DNA consisting of the nucleotide sequence of SEQ ID NO: 3;
(ii) DNA capable of hybridizing under stringent conditions to DNA consisting of a nucleotide sequence complementary to the nucleotide sequence of SEQ ID NO: 3, wherein a protein encoded thereby has a function of decreasing a copy number of an expression construct in the bacterium of the genus *Burkholderia,* or reducing stability thereof;
(iii) DNA consisting of a nucleotide sequence derived from the nucleotide sequence of SEQ ID NO: 3 by the deletion, substitution, or addition of one or more, preferably one or a few bases, wherein a protein encoded thereby has a function of decreasing a copy number of an expression construct in the bacterium of the genus *Burkholderia,* or reducing stability thereof;
(iv) DNA consisting of a nucleotide sequence having at least 80% or higher, preferably 90% or higher sequence homology, preferably sequence identity to SEQ ID NO: 3, wherein a protein encoded thereby has a function of decreasing a copy number of an expression construct in the bacterium of the genus *Burkholderia,* or reducing stability thereof;
(v) DNA consisting of a nucleotide sequence encoding a protein consisting of the amino acid sequence of SEQ ID NO: 4;
(vi) DNA capable of hybridizing under stringent conditions to DNA consisting of a nucleotide sequence encoding a protein consisting of the amino acid sequence of SEQ ID NO: 4, wherein a protein encoded thereby has a function of decreasing a copy number of an expression construct in the bacterium of the genus *Burkholderia,* or reducing stability thereof;
(vii) DNA consisting of a nucleotide sequence encoding a protein consisting of an amino acid sequence derived from the amino acid sequence of SEQ ID NO: 4 by the deletion, substitution, or addition of one or a few amino acids, wherein the protein has a function of decreasing a copy number of an expression construct in the bacterium of the genus *Burkholderia,* or reducing stability thereof; and
(viii) DNA consisting of a nucleotide sequence encoding a protein consisting of an amino acid sequence having at least 80% or higher, preferably 90% or higher sequence homology, preferably sequence identity to SEQ ID NO: 4, wherein the protein has a function of decreasing a copy number of an expression construct in the bacterium of the genus *Burkholderia,* or reducing stability thereof.

In a preferred aspect, the gene BSFP_068720 may comprise any one of the following DNAs (i) to (viii):
(i) DNA consisting of the nucleotide sequence of SEQ ID NO: 5;
(ii) DNA capable of hybridizing under stringent conditions to DNA consisting of a nucleotide sequence complementary to the nucleotide sequence of SEQ ID NO: 5, wherein a protein encoded thereby has a function of decreasing a copy number of an expression construct in the bacterium of the genus *Burkholderia,* or reducing stability thereof;
(iii) DNA consisting of a nucleotide sequence derived from the nucleotide sequence of SEQ ID NO: 5 by the deletion, substitution, or addition of one or more, preferably one or a few bases, wherein a protein encoded thereby has a function of decreasing a copy number of an expression construct in the bacterium of the genus *Burkholderia,* or reducing stability thereof;
(iv) DNA consisting of a nucleotide sequence having at least 80% or higher, preferably 90% or higher sequence homology, preferably sequence identity to SEQ ID NO: 5, wherein a protein encoded thereby has a function of decreasing a copy number of an expression construct in the bacterium of the genus *Burkholderia,* or reducing stability thereof;
(v) DNA consisting of a nucleotide sequence encoding a protein consisting of the amino acid sequence of SEQ ID NO: 6;
(vi) DNA capable of hybridizing under stringent conditions to DNA consisting of a nucleotide sequence encoding a protein consisting of the amino acid sequence of SEQ ID NO: 6, wherein a protein encoded thereby has a function of decreasing a copy number of an expression construct in the bacterium of the genus *Burkholderia,* or reducing stability thereof;
(vii) DNA consisting of a nucleotide sequence encoding a protein consisting of an amino acid sequence derived from the amino acid sequence of SEQ ID NO: 6 by the deletion, substitution, or addition of one or a few amino acids, wherein the protein has a function of decreasing a copy number of an expression construct in the bacterium of the genus *Burkholderia,* or reducing stability thereof; and
(viii) DNA consisting of a nucleotide sequence encoding a protein consisting of an amino acid sequence having at least 80% or higher, preferably 90% or higher sequence homology, preferably sequence identity to SEQ ID NO: 6, wherein the protein has a function of decreasing a copy number of an expression construct in the bacterium of the genus *Burkholderia,* or reducing stability thereof.

As used herein, the "stringent conditions" are, for example, conditions on the order of "1 x SSC, 0.1% SDS, and 37°C". More stringent conditions are conditions on the order of "0.5 x SSC, 0.1% SDS, and 42°C". Further stringent conditions are conditions on the order of "0.2 x SSC, 0.1% SDS, and 65°C". The isolation of DNA having higher homology, preferably higher identity, to a probe sequence can be expected with such increase in the stringency of hybridization conditions. However, the combinations of the SSC, SDS, and temperature conditions described above are a mere illustration, and those skilled in the art can achieve the same stringency as above by determining the stringency of hybridization or appropriately combining the above-described or other factors (e.g., a probe concentration, a probe length, and a hybridization reaction time). DNA that hybridizes under such stringent conditions generally has high sequence identity. The high sequence identity has at least 60% or higher, preferably 70% or higher, more preferably 80% or higher, particularly preferably 90% or higher, most preferably 95% or higher homology.

As used herein, the "one or more bases" differ in the number of bases depending on the full length of a reference sequence to be subjected to deletion, substitution, or addition, and means, for example, 1 to 30, preferably 1 to 20, more preferably 1 to 10 bases per nucleotide sequence consisting of 100 bases. Likewise, the "one or a few bases" are, for example, 1 to 10, preferably 1 to 5, more preferably 4, 3, 2, or 1 bases per nucleotide sequence consisting of 100 bases.

As used herein, the "one or more amino acids" differ in the number of amino acids depending on the full length of a reference sequence to be subjected to deletion, substitution, or addition, and means, for example, 1 to 30, preferably 1 to 20, more preferably 1 to 10 amino acids per amino acid sequence consisting of 100 amino acids. Likewise, the "one or a few amino acids" are, for example, 1 to 10, preferably 1 to 5, more preferably 4, 3, 2, or 1 amino acids per amino acid sequence consisting of 100 amino acids.

The sequence homology or the sequence identity is at least 80% or higher, preferably 90% or higher, more preferably 95% or higher, further preferably 98% or higher, to a particular sequence when calculated using BLAST or the like (e.g., using defaults, i.e., initially set parameters).

The protein encoded by a target gene, intended to be produced in the bacterium of the genus *Burkholderia* is also referred to as a "target protein". The target protein is not particularly limited as long as the protein can be produced in the bacterium of the genus *Burkholderia.* The target protein is preferably an extracellularly secretory protein which is difficult to express in a conventional host-vector system known in the art using E. *coli,* yeast/filamentous bacterium, or the like. Such a protein is an enzyme. Examples thereof include esterase, lipase, amylase, glucoamylase, α-galactosidase, β-galactosidase, α-glucosidase, β-glucosidase, mannosidase, isomerase, invertase, transferase, ribonuclease, deoxyribonuclease, chitinase, catalase, laccase, phenoloxidase, oxidase, oxide reductase, cellulase, xylanase, peroxidase, hydrolase, cutinase, protease, phytase, lyase, pectinase, aminopeptidase, and carboxypeptidase. Among these enzymes, esterase classified into EC 3.1.1 carboxylic-ester hydrolases is preferred, esterase classified into EC 3.1.1.1 carboxylesterase is more preferred, sterol esterase classified into EC 3.1.1.13 - sterol esterase or triacylglycerol lipase classified into EC 3.1.1.3 (lipase) is further preferred, and cholesterol esterase is particularly preferred.

Examples of the cholesterol esterase include sterol ester hydrolase derived from *Burkholderia stabilis* (EC3.1.1.13). Examples of the lipase include *Burkholderia glumae* lipase (L G Frenken et al., Mol Microbiol. 9 (3), 591-9 (1993)). The target protein may or may not comprise a signal peptide. The target protein preferably further comprises a signal peptide. For example, an E. *coli* signal peptide may be used as the signal peptide. A signal peptide reported in papers, etc. (Tullman-Ercek et al., J Bio Chem. 16, 282 (11), 8309-16 (2007)) or a signal peptide obtained by amino acid sequence search in a protein database Uniprot (http://www.uniprot.org/) may be used. Use of the latter method enables sequence information to be obtained on, for example, the signal peptide of a protein called BsmA (https://www.uniprot.org/uniprot/P39297), the signal peptide of a protein called YafY (https://www.uniprot.org/uniprot/P77365), and the signal peptide of a protein called YnjE (https://www.uniprot.org/uniprot/P78067) in E. *coli.*

A nucleotide sequence encoding the esterase is not particularly limited and may be, for example, a nucleotide sequence changed from the nucleotide sequence of any one of SEQ ID NOs: 11 to 13 according to the codon usage of a host such as a bacterium of the genus *Burkholderia* (e.g., *Burkholderia stabilis*). As long as the protein has the action of esterase, the nucleotide sequence may be a nucleotide sequence encoding a substantially equivalent amino acid sequence, or may be, for example, a nucleotide sequence encoding an equivalent amino acid sequence derived from the amino acid sequence of esterase by the mutation of some amino acids that are not involved in the action of esterase, for example, by the deletion, substitution, or addition of one or more such amino acids.

In a preferred aspect, a gene encoding the esterase may comprise, for example, any one of the following DNAs (i) to (iv):
(i) DNA consisting of a nucleotide sequence of any one of SEQ ID NOs: 11 to 13;
(ii) DNA capable of hybridizing under stringent conditions to DNA consisting of a nucleotide sequence complementary to a nucleotide sequence of any one of SEQ ID NOs: 11 to 13, wherein a protein encoded thereby has esterase activity;
(iii) DNA consisting of a nucleotide sequence derived from a nucleotide sequence of any one of SEQ ID NOs: 11 to 13 by the deletion, substitution, or addition of one or more, preferably one or a few bases, wherein a protein encoded thereby has esterase activity; and
(iv) DNA consisting of a nucleotide sequence having at least 80% or higher sequence identity to a nucleotide sequence of any one of SEQ ID NOs: 11 to 13, wherein a protein encoded thereby has esterase activity.

The target gene encoding the target protein is preferably linked to an expression construct such as a plasmid expressibly in the bacterium of the genus *Burkholderia.* The expression construct can be capable of replicating in the bacterium of the genus *Burkholderia.* Examples thereof include expression vectors, preferably autonomous replicating plasmid vectors. The expression vector is a plasmid vector capable of replicating in the bacterium of the genus *Burkholderia,* for example, a broad-host-range vector such as pBBR122, pSa, or pRK2.

The expression construct preferably comprises a promoter that controls the expression of the target gene. The promoter may be derived from the bacterium of the genus *Burkholderia.* The selection of a promoter having appropriate promoter activity increases the amount of the target gene transcribed and also increases the amount of the protein produced. Such a promoter is a promoter of BSFP_015180 gene encoding glycerol-3-phosphate dehydrogenase, wherein the promoter has promoter activity against a region of approximately 80 consecutive bp or more upstream of the start codon of the gene (hereinafter, also referred to as "14020 promoter"); or a promoter of BSFP_052240 gene encoding a functionally unknown protein, wherein the promoter has promoter activity against a region of approximately 100 consecutive bp or more upstream of the start codon of the gene (hereinafter, also referred to as "48230 promoter").

The expression construct can be constructed by those skilled in the art and may be constructed, for example, by integrating the promoter into any site of a plasmid vector replicating autonomously in the bacterium of the genus *Burkholderia,* and placing a multicloning site which facilitates the insertion of the target gen to be expressed or a terminator for terminating transcription, downstream of the promoter sequence.

The expression construct is allowed to replicate from a replication origin in a host. Hence, an expression construct having a replication origin that permits replication in a host is preferably used. An expression construct having a replication origin that permits replication in gram negative bacteria is preferred for gram negative bacteria including the bacterium of the genus *Burkholderia.* Examples of the plasmid vector having a replication origin that permits replication in gram negative bacteria include pBBR122 vector. pSa (Datta, American society for Microbiology, Washington, DC, pp. 9-15 (1975); Gorai et al., Plasmid 2 489-492 (1979); Ward and Grinsted, Plasmid 7 239-250 (1982); and Tait et al., Mol. Gen. Genet. 186 10-15 (1982)) or a plasmid having RK2ori (T. J. Schmidhauser et al., Plasmid. 9 (3) 325-30 (1983)), which has an initiation site of replication different from that of pBBR122, may also be used.

The expression construct can further comprise a selective marker and an origin of conjugation transfer. The bacterium of the genus *Burkholderia* can be transfected with the expression construct by an approach known to those skilled in the art. Examples thereof can include conjugation transfer and electroporation (Kim et al., Applied Biochemistry and Biotechnology, Vol. 73, 81-88 (1998)).

The target gene is inserted downstream of the promoter in the constructed expression construct, and host cells are transformed with the resulting plasmid vector. It can be expected that the target protein is constitutively expressed in a transformant selected according to the selective marker of the expression construct. The shuttle vector, the multicloning site, and the terminator used are not particularly limited. The expression construct also encompasses such a vector harboring the target gene to be expressed.

The target gene can be placed downstream of the promoter sequence, for example, at a position of 30 or less bases, preferably 15 or less bases, more preferably 6 or less bases from the promoter sequence, particularly preferably by direct linkage to the promoter sequence, under the control of the promoter sequence. The target gene may encode a signal sequence when the encoded protein is, for example, a secretory protein.

For example, a terminator sequence may not be placed downstream of the target gene and is preferably placed downstream thereof. The terminator sequence is a sequence that dissociates RNA polymerase from DNA to terminate transcription, and can usually be placed downstream of a gene. The terminator sequence is not particularly limited, and various terminator sequences known in the art can be used. When the target gene to be expressed encodes cholesterol esterase, a natural terminator sequence originally attached to the gene is preferably used because of its convenience.

Depending on the target protein, foldase or chaperon for expression, activation, or the like thereof may be co-expressed with the target protein. This forms a polypeptide chain having a correct three-dimensional structure and by extension, produces a protein having correct functions. When foldase or chaperon is required for protein production, the protein and the foldase or the chaperon are preferably co-expressed.

A nucleotide sequence encoding the chaperon is not particularly limited and may be, for example, a nucleotide sequence changed from a nucleotide sequence known in the art according to the codon usage of a host such as a bacterium of the genus *Burkholderia* (e.g., *Burkholderia stabilis*)*.* As long as the protein has functions as chaperon, the nucleotide sequence may be a nucleotide sequence encoding a substantially equivalent amino acid sequence, or may be, for example, a nucleotide sequence encoding an equivalent amino acid sequence derived from the amino acid sequence of chaperon by the mutation of some amino acids that are not involved in the functions of chaperon, for example, by the deletion, substitution, or addition of one or more such amino acids.

The foldase or the chaperon and the target protein to be co-expressed may be placed at sites distant from each other on a sequence and are preferably located downstream of a promoter necessary for the start of transcription.

For the co-expression of the foldase or chaperon gene with the target gene to be expressed, a foldase sequence or a chaperon sequence may be inserted between the target gene to be expressed and a terminator so that the foldase or chaperon gene is expressed under the promoter of the target gene to be expressed.

When the foldase or the chaperon functions at a particular site, a sequence encoding it preferably comprises a signal sequence.

In a preferred aspect, DNA encoding the foldase may comprise any one of the following DNAs (i) to (iv):
(i) DNA consisting of a nucleotide sequence of any one of SEQ ID NOs: 14 to 16;
(ii) DNA capable of hybridizing under stringent conditions to DNA consisting of a nucleotide sequence complementary to a nucleotide sequence of any one of SEQ ID NOs: 14 to 16, wherein a protein encoded thereby has foldase activity;
(iii) DNA consisting of a nucleotide sequence derived from a nucleotide sequence of any one of SEQ ID NOs: 14 to 16 by the deletion, substitution, or addition of one or more, preferably one or a few bases, wherein a protein encoded thereby has foldase activity; and
(iv) DNA consisting of a nucleotide sequence having at least 80% or higher, preferably 90% or higher sequence homology, preferably sequence identity to a nucleotide sequence of any one of SEQ ID NOs: 14 to 16, wherein a protein encoded thereby has foldase activity.

The target protein can be produced by expressing the target gene in the bacterium of the genus *Burkholderia.* Preferably, the bacterium of the genus *Burkholderia* transformed (i.e., a transformant) with the expression construct containing the gene encoding the target gene is cultured, and the protein encoded by the target gene can be isolated or purified from the cultures to produce the target protein. In this context, the cultures mean a mixture of bacterial cells and a solid or liquid culture solution obtained by the culture of the bacterium. The transformant can be cultured by a static culture method, a culture method using a roller bottle, or the like using a medium suitable for the host used. The culture can be performed by a method known in the art, and the medium or the culture conditions used can be appropriately determined depending on the type of the bacterial strain of the genus *Burkholderia* used.

When the target protein is produced within bacterial cells, the protein can be collected by homogenizing the bacterium of the genus *Burkholderia.* When the target protein is produced outside bacterial cells, the culture solution is directly used or the host cells are removed by centrifugation or the like. Then, the target protein can be isolated or purified from the cultures described above by using, either singly or in appropriate combination, general biochemical methods using various chromatographic techniques for use in protein isolation or purification.

The second embodiment provides 14020 promoter or 48230 promoter.

The 14020 promoter or the 48230 promoter can be widely used for the expression of a target gene in any bacterium of the genus *Burkholderia,* and exhibits markedly higher promoter activity when used in a bacterium of the genus *Burkholderia* whose gene on a particular chromosome is functionally inhibited than that when used in a wild-type strain.

The 14020 promoter may comprise any one of the following DNAs (i) to (iv):
(i) DNA that consists of a sequence of more than approximately 80 consecutive nucleotides, for example, 90 or more or 100 or more consecutive nucleotides, in the nucleotide sequence of SEQ ID NO: 7, and has promoter activity;
(ii) DNA that is capable of hybridizing under stringent conditions to DNA consisting of a complementary sequence of more than approximately 80 consecutive nucleotides, for example, 90 or more or 100 or more consecutive nucleotides, in the nucleotide sequence of SEQ ID NO: 7, and has promoter activity;
(iii) DNA that consists of a nucleotide sequence derived from a sequence of more than approximately 80 consecutive nucleotides, for example, 90 or more or 100 or more consecutive nucleotides, in the nucleotide sequence of SEQ ID NO: 7 by the deletion, substitution, or addition of one or more, preferably one or a few bases, and has promoter activity; and
(iv) DNA that consists of a nucleotide sequence having at least 80% or higher, preferably 90% or higher sequence homology, particularly preferably sequence identity to a sequence of more than approximately 80 consecutive nucleotides, for example, 90 or more or 100 or more consecutive nucleotides, in SEQ ID NO: 7, and has promoter activity.

In a preferred aspect, the 14020 promoter may comprise, for example, any one of the following DNAs (i) to (iv):
(i) DNA consisting of the nucleotide sequence of SEQ ID NO: 8;
(ii) DNA that is capable of hybridizing under stringent conditions to DNA consisting of a nucleotide sequence complementary to the nucleotide sequence of SEQ ID NO: 8, and has promoter activity;
(iii) DNA that consists of a nucleotide sequence derived from the nucleotide sequence of SEQ ID NO: 8 by the deletion, substitution, or addition of one or a few bases, and has promoter activity; and
(iv) DNA that consists of a nucleotide sequence having at least 80% or higher, preferably 90% or higher sequence homology, preferably sequence identity to SEQ ID NO: 8, and has promoter activity.

The 48230 promoter may comprise any one of the following DNAs (i) to (iv):
(i) DNA that consists of a sequence of more than approximately 100 consecutive nucleotides, for example, 110 or more or 120 or more consecutive nucleotides, in the nucleotide sequence of SEQ ID NO: 9, and has promoter activity;
(ii) DNA that is capable of hybridizing under stringent conditions to DNA consisting of a complementary sequence of more than approximately 100 consecutive nucleotides, for example, 110 or more or 120 or more consecutive nucleotides, in the nucleotide sequence of SEQ ID NO: 9, and has promoter activity;
(iii) DNA that consists of a nucleotide sequence derived from a sequence of more than approximately 100 consecutive nucleotides, for example, 110 or more or 120 or more consecutive nucleotides, in the nucleotide sequence of SEQ ID NO: 9 by the deletion, substitution, or addition of one or more, preferably one or a few bases, and has promoter activity; and
(iv) DNA that consists of a nucleotide sequence having at least 80% or higher, preferably 90% or higher sequence homology, preferably sequence identity to a sequence of more than approximately 100 consecutive nucleotides, for example, 110 or more or 120 or more consecutive nucleotides, in SEQ ID NO: 9, and has promoter activity.

In a preferred aspect, the 48230 promoter may comprise, for example, any one of the following DNAs (i) to (iv):
(i) DNA consisting of the nucleotide sequence of SEQ ID NO: 10;
(ii) DNA that is capable of hybridizing under stringent conditions to DNA consisting of a nucleotide sequence complementary to the nucleotide sequence of SEQ ID NO: 10, and has promoter activity;
(iii) DNA that consists of a nucleotide sequence derived from the nucleotide sequence of SEQ ID NO: 10 by the deletion, substitution, or addition of one or a few bases, and has promoter activity; and
(iv) DNA that consists of a nucleotide sequence having at least 80% or higher, preferably 90% or higher sequence homology, preferably sequence identity to SEQ ID NO: 10, and has promoter activity.

The third embodiment provides a mutant strain of a bacterium of the genus *Burkholderia* engineered to have inhibited expression of one or more genes selected from the group consisting of genes on a particular chromosome, for example, BSFP_068740, BSFP_068730, and BSFP_068720, or proteins encoded by the genes.

The bacterium of the genus *Burkholderia* used may occur naturally or may be artificially created as long as a gene having a function of eliminating the stability of an expression vector such as a plasmid, residing on the particular chromosome is functionally inhibited.

In a preferred aspect, the bacterium is engineered to have a disrupted gene on the particular chromosome or have inhibited expression of the gene or a protein encoded by the gene.

An approach known in the art to delete functions of the gene of interest can be used in such inhibition. Examples thereof include gene disruption by gene recombination, and the induction of loss of function by mutagenesis. The desired mutation may be caused as to an existing natural or mutant bacterium of the genus *Burkholderia* by treatment with a mutagen such as nitrosoguanidine or physical treatment such as irradiation with ultraviolet ray, x ray, or gamma ray. Also, the expression of the gene of interest may be inhibited by deleting a promoter region upstream of the gene of interest or deleting functions of the promoter.

A method that is carried out for *Burkholderia cenocepacia* (Agnoli et al., Mol Microbiol., 83 (2), 362-378 (2012)) or a method that is carried out for *Burkholderia ubonensis* (Price et al., PLoS Negl Trop Dis.. 11 (9): e0005928 (2017)) may be used as a method for deleting the chromosome itself.

Examples of microbes classified into the genus *Burkholderia* include *Burkholderia stabilis* mentioned above as well as *Burkholderia multivorans, Burkholderia cepacia, Burkholderia mallei, Burkholderia glumae, Burkholderia ambifaria, Burkholderia dolosa, Burkholderia gladioli,* and *Burkholderia plantarii* (AZEGAMI et al., Int J Syst Evol Microbiol, 37 (2): 144-152 (1987); and Seo et al., BMC Genomics, 16: 346 (2015)). *Burkholderia stabilis* or *Burkholderia silvatlantica* is preferred.

Since the bacterium of the genus *Burkholderia* exists in various natural ecosystems, the medium or the conditions for culturing the bacterium of the genus *Burkholderia* vary. For example, in order to culture *Burkholderia stabilis,* the bacterium can be cultured at 30°C in LB medium (1% Bacto(TM) Tryptone, 0.5% Bacto(TM) Yeast extract, and 0.5% sodium chloride). As for bacterial strains purchasable at DSMZ, media and conditions recommended by DSMZ are published as cultivation conditions and can be referred to.

The fourth embodiment provides a method for maintaining an expression construct in a bacterium of the genus *Burkholderia,* comprising the step of culturing a bacterium of the genus *Burkholderia,* wherein the bacterium of the genus *Burkholderia* lacks one or more genes selected from the group consisting of genes on a particular chromosome, for example, BSFP_068740, BSFP_068730, and BSFP_068720, or has inhibited expression of the genes or proteins encoded by the genes, and the bacterium comprises the expression construct.

In a preferred aspect, the cultured bacterium of the genus *Burkholderia* can have an increased copy number of the expression construct or increased stability thereof as compared with a bacterium of the genus *Burkholderia* whose gene on the particular chromosome is not functionally inhibited.

Such an increased copy number or stability of the expression construct enables a target protein to be highly expressed. The stability of the expression construct can be evaluated by using, as an index, the stability of the expression construct in a host, for example, the number of colonies proliferated on a medium containing an antibiotic based on the number of colonies proliferated on a medium containing no antibiotic (expression construct retention ratio).

The fifth embodiment provide a method for increasing the stability of an expression construct in a bacterium of the genus *Burkholderia,* comprising the step of culturing a bacterium of the genus *Burkholderia,* wherein the bacterium of the genus *Burkholderia* lacks one or more genes selected from the group consisting of genes on a particular chromosome, for example, BSFP_068740, BSFP_068730, and BSFP_068720, or has inhibited expression of the genes or proteins encoded by the genes, and the bacterium comprises the expression construct.

### Examples

Hereinafter, the present invention will be described with reference to Examples, etc. However, the scope of the present invention is not construed as being limited by Examples, etc. given below. Measurement values, etc. given below may vary depending on measurement conditions, the precision of equipment used, and the like. In agarose gel electrophoresis in Examples, 1 Kb plus DNA ladder marker RTU manufactured by GeneDireX, Inc. was used as a molecular weight marker. This marker consists of 13 fragments, and their molecular weights are 100 bp, 250 bp, 500 bp, 750 bp, 1000 bp, 1500 bp, 2000 bp, 3000 bp, 4000 bp, 5000 bp, 6000 bp, 8000 bp, and 10000 bp in order from the smallest one.

### Example 1 Preparation of gene disrupted strain (homologous recombination)

*Burkholderia stabilis* (deposition No: NITE BP-02704) was shake-cultured at 30°C in LB medium (1% Bacto(TM) Tryptone, 0.5% Bacto(TM) Yeast extract, and 0.5% sodium chloride) and thereby proliferated until a stationary phase. 500 µL of the resulting bacterial solution was added to 10 mL of LB medium and cultured at 30°C for 12 hours.

The bacterium thus cultured was recovered by centrifugation (3,000 g, 10 min, room temperature), and total DNA was extracted with DNeasy(R) Blood & Tissue Kit (manufactured by Qiagen N.V.).

DNA amplification was performed by polymerase chain reaction (hereinafter, abbreviated to PCR: Saiki et al., Science, 239 487-491 (1988)) using the obtained total DNA of *Burkholderia stabilis* as a template and synthetic oligodeoxyribonucleotide primers (hereinafter, abbreviated to primers) of SEQ ID NOs: 17 and 18. The enzyme for PCR used was KOD FX Neo (manufactured by Toyobo Co., Ltd.). As a result, DNA consisting of SEQ ID NO: 19 was obtained. These DNA fragments amplified by PCR reaction were subjected to agarose gel electrophoresis, excised from the gel, and purified using QIAquick Gel Extraction Kit (manufactured by Qiagen N.V.).

DNA amplification was performed by PCR in the same manner as above using pK18mobsacB (SCHAFER et al., Gene, 145 (1) 69-73 (1994)) as a template and primers of SEQ ID NOs: 20 and 21. As a result, DNA consisting of approximately 4.1 kb was obtained. The DNA fragments amplified by PCR reaction were purified by agarose gel electrophoresis in the same manner as above.

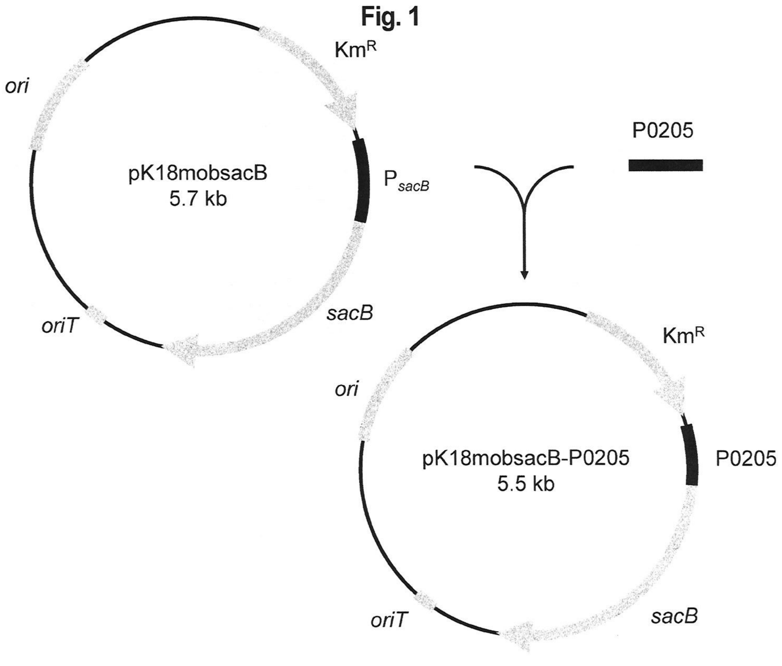

The respective purified DNA fragments consisting of SEQ ID NO: 19 and approximately 4.1 kb were linked using In-Fusion(R) HD Cloning Kit (manufactured by Takara Bio Inc.), and E. *coli* DH5a was transformed therewith and then spread over LB agar medium containing 50 µg/mL kanamycin. After culture at 30°C for 24 hours, the obtained colonies were inoculated to LB medium containing 50 µg/mL kanamycin and cultured at 37°C for 12 hours, followed by plasmid extraction from the culture solution using QIAprep Spin Miniprep Kit (manufactured by Qiagen N.V.) to obtain pK18mobsacB-P0205 for disruptant preparation (Figure 1).

DNA amplification was performed by PCR using the total DNA of *Burkholderia stabilis* as a template and primers of SEQ ID NOs: 22 and 23, SEQ ID NOs: 24 and 25, SEQ ID NOs: 26 and 27, SEQ ID NOs: 28 and 29, SEQ ID NOs: 30 and 31, or SEQ ID NOs: 32 and 33. The DNA fragments amplified by PCR reaction were purified by agarose gel electrophoresis in the same manner as above to obtain each DNA sequence. The DNA sequences thus obtained were designated as BSFP_068720A, BSFP_068720B, BSFP_068730A, BSFP_068730B, BSFP_068740A, and BSFP_068740B, respectively.

pK18mobsacB-P0205 was cleaved with EcoRI and HindIII (manufactured by Takara Bio Inc.) and purified by agarose gel electrophoresis. Three 3 fragments, the obtained pK18mobsacB-P0205 fragment, BSFP_068720A, and BSFP_068720B, were linked using In-Fusion(R) HD Cloning Kit (manufactured by Takara Bio Inc.), and E. *coli* DH5a was transformed therewith. Likewise, three fragments, the pK18mobsacB-P0205 fragment, BSFP_068730A, and BSFP_068730B, or three fragments, the pK18mobsacB-P0205 fragment, BSFP_068740A, and BSFP_068740B, were linked using In-Fusion(R) HD Cloning Kit (manufactured by Takara Bio Inc.), and E. *coli* DH5a was transformed therewith. The obtained E. *coli* containing each of three transformants was spread over LB agar medium containing 50 µg/mL kanamycin. After culture at 30°C for 24 hours, the obtained colonies were inoculated to LB medium containing 50 µg/mL kanamycin and cultured at 37°C for 12 hours, followed by plasmid extraction from the culture solution to obtain pK18mobsacB-P0205-BSFP_068720, pK18mobsacB-P0205-BSFP_068730, and pK18mobsacB-P0205-BSFP_068740 which were vectors for disruptions.

E. *coli* DH5a was transformed with each vector obtained to obtain DH5α(pK18mobsacB-P0205-BSFP_068720), DH5α(pK18mobsacB-P0205-BSFP_068730), and DH5α(pK18mobsacB-P0205-BSFP_068740). *E. coli* HB101 was transformed with a helper plasmid pRK2013 (ATCC37159) to obtain HB101(pRK2013).

*Burkholderia stabilis* was inoculated to 5 mL of LB medium and cultured at 30°C for 12 hours to obtain a *Burkholderia stabilis* culture solution. Also, DH5α(pK18mobsacB-P0205-BSFP_068720), DH5α(pK18mobsacB-P0205-BSFP_068730), DH5α(pK18mobsacB-P0205-BSFP_068740), or HB101(pRK2013) was inoculated to 10 mL of LB medium containing 20 µg/mL kanamycin and cultured at 37°C for 12 hours to obtain each *E*. *coli* culture solution.

1.5 mL of the *Burkholderia stabilis* culture solution was centrifuged (15,000 g, 1 min, 4°C) to obtain *Burkholderia stabilis* bacterial cells.

Also, 2 mL of the DH5α(pK18mobsacB-P0205-BSFP_068720), DH5α(pK18mobsacB-P0205-BSFP_068730), DH5α(pK18mobsacB-P0205-BSFP_068740), or HB101(pRK2013) culture solution was centrifuged (15,000 g, 1 min, 4°C), and the supernatant was removed. 2 mL of LB medium was added for suspension, and the bacterial cells were centrifuged (15,000 g, 1 min, 4°C) again to obtain DH5α(pK18mobsacB-P0205-BSFP_068720), DH5α(pK18mobsacB-P0205-BSFP_068730), DH5α(pK18mobsacB-P0205-BSFP_068740), and HB101(pRK2013) bacterial cells.

The *Burkholderia stabilis* bacterial cells, the DH5α(pK18mobsacB-P0205-BSFP_068720) bacterial cells, and the HB101(pRK2013) bacterial cells were suspended and mixed in 100 µL of LB medium and spread over LB agar medium. Likewise, a combination of the *Burkholderia stabilis* bacterial cells, the DH5α(pK18mobsacB-P0205-BSFP_068730) bacterial cells, and the HB101(pRK2013) bacterial cells, or a combination of the *Burkholderia stabilis* bacterial cells, the DH5α(pK18mobsacB-P0205-BSFP_068740) bacterial cells, and the HB101(pRK2013) bacterial cells was suspended and spread over LB agar medium. Each agar medium was cultured overnight at 30°C.

After a lapse of overnight, the grown bacterial cells of each combination were scraped with a bacteria spreader and suspended in 1 mL of a 10 mM magnesium sulfate solution. The suspension was diluted 10-fold, and 100 µL of the dilution was inoculated to Nutrient Broth (manufactured by Becton, Dickinson and Company) agar medium containing 200 µg/mL kanamycin and 50 µg/mL ampicillin and cultured at 30°C for 2 days to obtain single recombinants for BSFP_068720, BSFP_068730, or BSFP_068740 disruptions.

Subsequently, each single recombinant was inoculated to 5 mL of LB medium and cultured at 30°C for 12 hours. The obtained culture solution was diluted 10³-to 10⁵-fold with LB medium, inoculated to an agar medium containing 0.33% Bacto(TM) Tryptone, 0.17% Bacto(TM) Yeast extract, 0.5% sodium chloride, 10% sucrose, and 1.5% agar, and cultured at 30°C for 2 days.

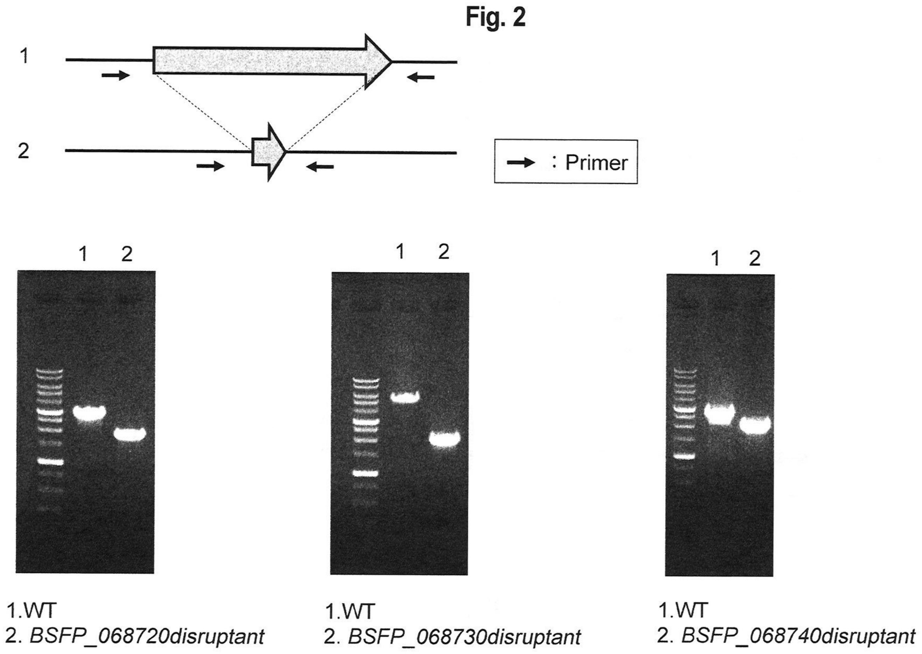

The obtained colonies were inoculated to Nutrient Broth agar medium and Nutrient Broth agar medium containing 200 µg/mL kanamycin to select colonies that lost kanamycin resistance. PCR was carried out using primers described in SEQ ID NOs: 34 and 35 for the colonies derived from BSFP_068720 single recombination, primers described in SEQ ID NOs: 36 and 37 for the colonies derived from BSFP_068730 single recombination, and primers described in SEQ ID NOs: 38 and 39 for the colonies derived from BSFP_068740 single recombination. Agarose gel electrophoresis was carried out to confirm that each gene was truncated and was no longer able to function because the length was shorter as compared with the wild-type strain (Figure 2). These strains were designated as a BSFP_068720 disruptant, a BSFP_068730 disruptant, and a BSFP_068740 disruptant, respectively.

### Example 2 Preparation of gene disruptant (mutagenesis)

*Burkholderia stabilis* was inoculated to 100 mL of LB medium and cultured at 28°C for 20 hours. 30 mL of the culture solution was centrifuged, and the supernatant was removed to obtain bacterial cells. The bacterial cells were suspended by the addition of 30 mL of a 0.85% aqueous sodium chloride solution. To 9.5 mL of the suspended bacterial cells, 0.5 mL of a Tris-maleate buffer solution (2 M Tris and 2 M maleate, pH 6.2) was added, then 50 mg/mL 1-methyl-3-nitro-1-nitrosoguanidine and 1 mL of N,N-dimethylformamide were added, and the bacterial cells were shaken at 200 rpm at 28°C for 60 minutes. The bacterial cells thus shaken were centrifuged, and the supernatant was removed. The obtained bacterial cells were suspended by the addition of 10 mL of a 0.85% aqueous sodium chloride solution. The bacterial cells obtained by centrifugation and removal of the supernatant in the same manner as above were suspended by the addition of 10 mL of a 0.85% aqueous sodium chloride solution and centrifuged again to obtain bacterial cells. 10 mL of LB medium was added to the bacterial cells, which were then shake-cultured at 200 rpm at 28°C for 2 hours. 100 µL each of the bacterial cells diluted into varying concentrations was inoculated to LB agar medium and cultured at 28°C for 48 hours.

The colonies were suspended in 20 µL of Buffer P1 of QIAprep Spin Miniprep Kit (manufactured by Qiagen N.V.), and 20 µL of Buffer P2 was added to the suspension. DNA amplification was performed by PCR using the obtained solution as a template and primers of SEQ ID NOs: 40 and 41. The enzyme for PCR used was KOD FX Neo (manufactured by Toyobo Co., Ltd.). A strain that was not amplified with this set of primers was shake-cultured at 30°C in LB medium and thereby proliferated until a stationary phase. 500 µL of the resulting bacterial solution was added to 10 mL of LB medium and cultured at 30°C for 12 hours. The bacterial cells thus cultured were recovered by centrifugation and resuspended in 500 µL of Tris-EDTA (TE) buffer, and 50 µL of proteinase K (20 mg/mL) and 25 µL of 10% SDS were added to the suspension and incubated at 55°C for 30 minutes. After reaction, 600 µL of a mixed solution of phenol, chloroform, and isoamyl alcohol (50:49:1) was added thereto, stirred, and centrifuged. The obtained supernatant was transferred to another tube, followed by re-extraction with a mixed solution of phenol, chloroform, and isoamyl alcohol. Then, DNA was precipitated by the addition of 50 µL of 3 M sodium acetate and 350 µL of isopropanol to the obtained supernatant, and recovered DNA was washed twice with 500 µL of 70% ethanol. The DNA was dried in air at room temperature for 10 minutes and dissolved in 200 µL of deionized water. The obtained DNA was subjected to sequence library preparation in accordance with the standard protocol (350 bp Insert) of TruSeq DNA PCR-Free Library Prep Kit (manufactured by Illumina, Inc.). Subsequently, DNAseq was performed using a next-generation sequencer HiSEq under conditions of paired end and 100 bases/read. The absence of chromosome 3 was confirmed from an output read sequence. This strain that underwent DNAseq was designated as a chromosome 3 deletion mutant.

### Example 3 Preparation of cholesterol esterase expression vector

A cholesterol esterase expression vector described in Japanese Patent Laid-Open No. 2019-205402 (supra) was prepared by the following method. DNA amplification was performed by PCR using the *Burkholderia stabilis* total DNA obtained in Example 1 as a template and primers of SEQ ID NOs: 42 and 43, SEQ ID NOs: 44 and 45, or SEQ ID NOs: 46 and 47. As a result, two types of terminators (SEQ ID NOs: 48 and 49) and a multicloning site (BglII, NheI, SnaBI, SacI, KpnI, SpeI, BamHI, XbaI, and NdeI) fragment (SEQ ID NO: 50) were obtained. Likewise, DNA amplification was performed using pBBR122 plasmid (manufactured by MoBiTec GmbH) as a template and primers of SEQ ID NOs: 51 and 52. These DNA fragments amplified by PCR reaction were subjected to agarose gel electrophoresis, excised from the gel, and purified using QIAquick Gel Extraction Kit (manufactured by Qiagen N.V.). The purified DNA fragments were linked using InFusion(R) HD Cloning Kit (manufactured by Takara Bio Inc.), and *E*. *coli* DH5α was transformed therewith and then spread over LB agar medium containing 50 µg/mL kanamycin. After culture at 30°C for 24 hours, the obtained colonies were inoculated to LB medium containing 50 µg/mL kanamycin and cultured at 37°C for 12 hours, followed by plasmid extraction from the culture solution using QIAprep Spin Miniprep Kit (manufactured by Qiagen N.V.) to obtain pBBR122-T-MCS-T.

DNA amplification was performed by PCR using the *Burkholderia stabilis* total DNA obtained in Example 1 as a template and primers of SEQ ID NOs: 53 and 54 or SEQ ID NOs: 55 and 56 in the sequence listing. As a result, a DNA fragment of a gene encoding cholesterol esterase and its chaperon, and a DNA fragment of a promoter region were obtained. The obtained DNA fragments were subjected to agarose gel electrophoresis, excised from the gel, and purified using QIAquick Gel Extraction Kit (manufactured by Qiagen N.V.). DNA amplification was performed using pBBR122-T-MCS-T as a template and primers of SEQ ID NOs: 57 and 58 in the sequence listing to amplify a DNA fragment of the expression vector. The obtained expression vector DNA fragment and the preceding purified DNAs of the DNA fragment of a gene encoding cholesterol esterase and its chaperon and the DNA fragment of a promoter region were linked using In-Fusion(R) HD Cloning Kit (manufactured by Takara Bio Inc.). *E. coli* DH5α was transformed with the linked DNA and then spread over LB agar medium containing 50 µg/mL kanamycin. After culture at 30°C for 24 hours, the obtained colonies were inoculated to LB medium containing 50 µg/mL kanamycin and cultured at 37°C for 12 hours, followed by plasmid extraction from the culture solution using QIAprep Spin Miniprep Kit (manufactured by Qiagen N.V.) to obtain pBBR122-182.

### Example 4 Preparation of recombinant

The *Burkholderia stabilis* wild-type strain and the BSFP_068720 disruptant, the BSFP_068730 disruptant, the BSFP_068740 disruptant, and the chromosome 3 deletion mutant obtained in Examples 1 and 2 were each shake-cultured at 30°C in 100 mL of LB medium until reaching a logarithmic growth phase. Then, bacterial cells were recovered by centrifugation. The recovered bacterial cells were suspended by the addition of 100 mL of ice-cold sterilized water and then centrifuged to recover bacterial cells. This operation was repeated once. Then, the recovered bacterial cells were well suspended by the addition of 5 mL of a cold 10% glycerin solution and centrifuged to recover bacterial cells.

The recovered bacterial cells were suspended by the addition of 5 mL of an ice-cold 10% glycerin solution, dispensed in 40 µL portions, and frozen at - 80°C to obtain respective competent cells of the wild-type strain, the BSFP_068720 disruptant, the BSFP_068730 disruptant, the BSFP_068740 disruptant, and the chromosome 3 deletion mutant. The obtained competent cells of each strain were thawed on ice, and pBBR122 and the pBBR122-182 obtained in Example 3 were each added thereto in an amount of approximately 200 to 400 ng and mixed. This mixed solution was transferred to a 0.2 cm wide electroporation cuvette (manufactured by Bio-Rad Laboratories, Inc.) and given electric pulse at an electric field intensity of 12.5 kV/cm, a capacitance of 25 µF, and an external resistance of 200 Ω using a gene transduction apparatus Gene Pulser II. The mixed solution of the bacterial cells and the DNA treated with electric pulse was mixed with 1 mL of LB medium and cultured at 30°C for 1 hour. Then, 200 µL of the bacterial solution was spread over LB medium containing 50 µg/mL kanamycin and cultured at 30°C for 1 day to obtain a transformant of each expression vector, i.e., wild-type strain-pBBR122, wild-type strain-pBBR122-182, BSFP_068720 disruptant-pBBR122, BSFP_068720 disruptant-pBBR122-182, BSFP_068730 disruptant-pBBR122, BSFP_068730 disruptant-pBBR122-182, BSFP_068740 disruptant-pBBR122, BSFP_068740 disruptant-pBBR122-182, chromosome 3 deletion mutant-pBBR122, and chromosome 3 deletion mutant-pBBR122-182.

### Example 5 Confirmation of copy number of plasmid

The transformants wild-type strain-pBBR122, BSFP_068720 disruptant-pBBR122, BSFP_068730 disruptant-pBBR122, BSFP_068740 disruptant-pBBR122, and chromosome 3 deletion mutant-pBBR122 obtained in Example 4 were each inoculated to 5 mL of LB medium containing 100 µg/mL kanamycin and cultured at 28°C for 12 hours. The resulting bacterial solution was added at a bacterial cell turbidity of OD = 0.2 to 5 mL of LB medium containing 100 µg/mL kanamycin and cultured at 28°C for 24 hours or 48 hours to obtain bacterial cells of each strain differing in culture time. The bacterial cells cultured for 24 hours and the bacterial cells cultured for 48 hours were each recovered by centrifugation (3,000 g, 10 min, room temperature).

Total DNA was extracted from the bacterial cells cultured for 24 hours using QIAamp DNA Mini Kit (manufactured by Qiagen N.V.).

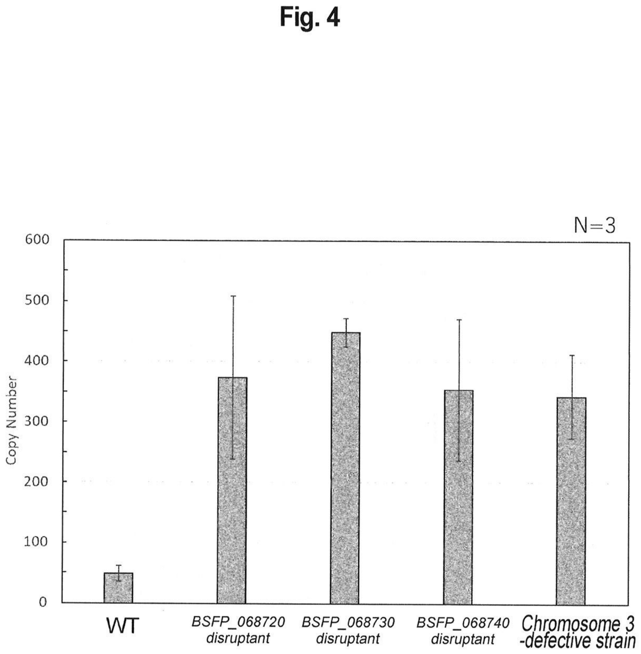

In order to confirm the copy number of each plasmid, each total DNA was subjected to real-time PCR by the method described in Choi KH et al., Appl. Environ. Microbiol., 74 (4) 1064-75 (2008). Specifically, aspartate-semialdehyde dehydrogenase gene was amplified as a chromosomal gene using a primer set of SEQ ID NOs: 59 and 60. The obtained DNA fragments were subjected to agarose gel electrophoresis, excised from the gel, and purified using QIAquick Gel Extraction Kit (manufactured by Qiagen N.V.) to obtain a DNA fragment. This DNA fragment and a DNA fragment obtained by the cleavage of pBBR122 with Smal (manufactured by Takara Bio Inc.) were linked using In-Fusion(R) HD Cloning Kit (manufactured by Takara Bio Inc.). *E. coli* DH5α was transformed with the linked DNA and then spread over LB agar medium containing 50 µg/mL chloramphenicol. After culture at 30°C for 24 hours, the obtained colonies were inoculated to LB medium containing 50 µg/mL chloramphenicol and cultured at 37°C for 12 hours, followed by plasmid extraction from the culture solution using QIAprep Spin Miniprep Kit (manufactured by Qiagen N.V.) to obtain BBR122-asd. The total DNA, pBBR122-asd, and each primer set (a primer set of SEQ ID NOs: 61 and 62 for chromosome copy number calculation, and a primer set of SEQ ID NOs: 63 and 64 for plasmid copy number calculation) were mixed in accordance with the protocol of LightCycler FastStart DNA Master Plus SYBR Green I (manufactured by F. Hoffmann-La Roche Ltd.) to prepare a sample. Real-time PCR was carried out using LightCycler 480 (manufactured by F. Hoffmann-La Roche Ltd.). The PCR was carried out under conditions involving denaturation at 95°C for 10 seconds, annealing at 60°C for 30 seconds, and extension at 72°C for 1 second. A Cp value calculated by the 2nd derivative maximum method was output as to each gene. A calibration curve was prepared using the Cp value of pBBR122-asd, and the copy number of the plasmid of each strain was calculated (Figure 4). As a result, pBBR122 in the wild-type strain had approximately 30 copies, whereas pBBR122 in the BSFP_068720 disruptant, the BSFP_068730 disruptant, the BSFP_068740 disruptant, and the chromosome 3 deletion mutant had approximately 300 to 400 copies, indicating that functional deletion of at least any one of BSFP_068720, BSFP_068730, and BSFP_068740 increases the copy number of a plasmid.

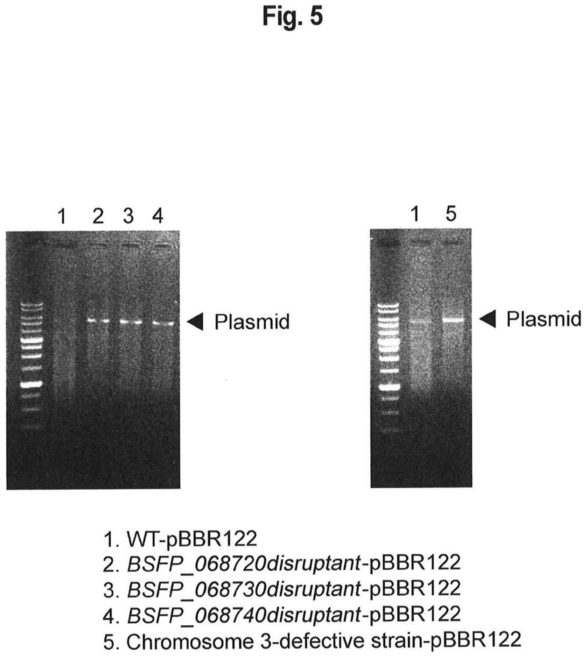

The plasmid was extracted from the bacterial cells cultured for 48 hours using QIAprep Spin Miniprep Kit (manufactured by Qiagen N.V.). The obtained plasmid was cleaved with XhoI (manufactured by Takara Bio Inc.) and subjected to agarose gel electrophoresis (Figure 5). As a result, a single signal derived from the plasmid was seen, and the signal was stronger in BSFP_068720 disruptant-pBBR122, BSFP_068730 disruptant-pBBR122, BSFP_068740 disruptant-pBBR122, and chromosome 3 deletion mutant-pBBR122 compared with wild-type strain-pBBR122.

These results indicated that a signal obtained in agarose electrophoresis was stronger in the BSFP_068720, BSFP_068730, or BSFP_068740 disruptant transfected with pBBR122 than in the wild-type strain transfected with pBBR122 and the copy number of the plasmid in the real-time PCR results was also larger in these disruptants. The results also indicated that the copy number of the plasmid was also larger in the mutant with chromosome 3 including BSFP_068720, BSFP_068730, and BSFP_068740 being deleted, transfected with pBBR122 than in the wild-type strain transfected with pBBR122. This also indicated that functional deletion of at least any one of BSFP_068720, BSFP_068730, and BSFP_068740 increases the copy number of a plasmid.

### Example 6 Confirmation of stability of plasmid

The transformants wild-type strain-pBBR122-182, BSFP_068720 disruptant-pBBR122-182, BSFP_068730 disruptant-pBBR122-182, BSFP_068740 disruptant-pBBR122-182, and chromosome 3 deletion mutant-pBBR122-182 obtained in Example 4 were each inoculated to 5 mL of LB medium and cultured at 28°C for 24 hours. The culture solution obtained 24 hours later was regarded as the first generation, and 2 µL of the first-generation culture solution was inoculated to 5 mL of fresh LB medium and cultured at 28°C for 24 hours. The obtained culture solution was regarded as the second generation, and 2 µL of the second-generation culture solution was inoculated to 5 mL of fresh LB medium and cultured at 28°C for 24 hours. The obtained culture solution was regarded as the third generation. The first-generation culture solution and the third-generation culture solution were each diluted with 10⁷-fold with LB medium and inoculated to LB agar medium and LB agar medium containing 100 µg/mL kanamycin. The number of colonies grown by culture at 30°C for 2 days was measured and regarded as a colony forming unit (CFU). Colonies grown in LB agar medium represent a total bacterial count, and colonies grown in LB medium containing kanamycin represent a plasmid-retaining bacterial count. Therefore, the plasmid retention ratio was calculated according to the following expression. Plasmid retention ratio (%) = CFU in LB agar medium containing kanamycin / CFU in LB agar medium × 100

**[Table 1]**

| | First generation | | | Third generation | | |
|---|---|---|---|---|---|---|
| | Km(-) | Km(+) | Plasmid retention ratio (%) | Km(-) | Km(+) | Plasmid retention ratio (%) |
| Wild-type strain-pBBR122-182 | 145 | 0 | 0 | 138 | 0 | 0 |
| ΔBSFP_068720-pBBR122-182 | 92 | 111 | 121 | 91 | 89 | 98 |
| ΔBSFP_068730-pBBR122-182 | 103 | 81 | 79 | 90 | 85 | 94 |
| ΔBSFP_068740-pBBR122-182 | 88 | 97 | 110 | 104 | 76 | 73 |
| Chromosome 3 deletion mutant-pBBR122-182 | 100 | 90 | 90 | 88 | 82 | 93 |

The numeric values in the table represent CFU of the culture solutions diluted 10⁷-fold. Km(-) in the table represents CFU in LB agar medium, and Km(+) represents CFU in LB agar medium containing 100 µg/mL kanamycin.

As shown in Table 1, the wild-type strain containing the expression vector lost the plasmid once cultured in LB liquid medium containing no kanamycin. On the other hand, the BSFP_068720, BSFP_068730, or BSFP_068740 disruptant or the mutant with chromosome 3 including these three genes being deleted became less likely to lose the plasmid even when passaged three times in LB medium containing no kanamycin. These results indicated that functional deletion of at least any one of BSFP_068720, BSFP_068730, and BSFP_068740 allows a plasmid to be stably retained in bacterial cells.

### Example 7 Confirmation of cholesterol esterase productivity

Wild-type strain-pBBR122-182, BSFP_068720 disruptant-pBBR122-182, BSFP_068730 disruptant-pBBR122-182, BSFP_068740 disruptant-pBBR122-182, and chromosome 3 deletion mutant-pBBR122-182 among the transformants obtained in Example 4 were each inoculated to 5 mL of YSO medium (3% yeast extract, 3% sorbitol, and 3% methyl oleate, pH 7.0) containing 100 µg/mL kanamycin and cultured at 28°C for 72 hours. The amount of cholesterol esterase produced by each strain thus cultured was evaluated by use of the following enzymatic activity measurement method.

### <Enzymatic activity measurement method>

A reaction reagent containing the following reagents at the following concentrations was prepared.

### [Reaction reagent]

40 mM potassium phosphate buffer solution (pH 6.8)
0.02% TODB (Dojindo Laboratories Co., Ltd.)
10 U/mL peroxidase
0.3% Triton X-100
2.5 U/mL cholesterol oxidase
10% fetal bovine serum solution
0.035% 4-aminoantipyrine

The reaction reagent was preliminarily warmed at 37°C for 10 minutes, and 3 µL of a recombinant cholesterol esterase solution was added to 150 µL of the reaction reagent using Hitachi 7080 automatic analyzer (manufactured by Hitachi, Ltd.) and reacted at 37°C. Absorbance was measured at a main wavelength of 546 nm and a sub wavelength of 660 nm from 76.69 seconds to 119.53 seconds after addition to obtain the amount of change in absorbance per unit time (ΔA/min). A value obtained according to the following expression was defined as cholesterol esterase activity, and the cholesterol esterase activity was calculated. Cholesterol esterase activity (U/mL) = ΔA / min / 18 x (150 + 3) / 3 / 1000 wherein ΔA/min represents the amount of change in absorbance per unit time obtained by measuring absorbance at a main wavelength of 546 nm and a sub wavelength of 660 nm from 76.69 seconds to 119.53 seconds after addition of the reaction reagent; 18 represents the millimolar molecular extinction coefficient at 550 nm of an oxidative condensation reaction product of TODB and 4-aminoantipyrine; 150 + 3 represents the total reaction volume (the volume of the reaction reagent + the volume of the recombinant lipase solution); and 3 represents the volume of the recombinant cholesterol esterase solution.

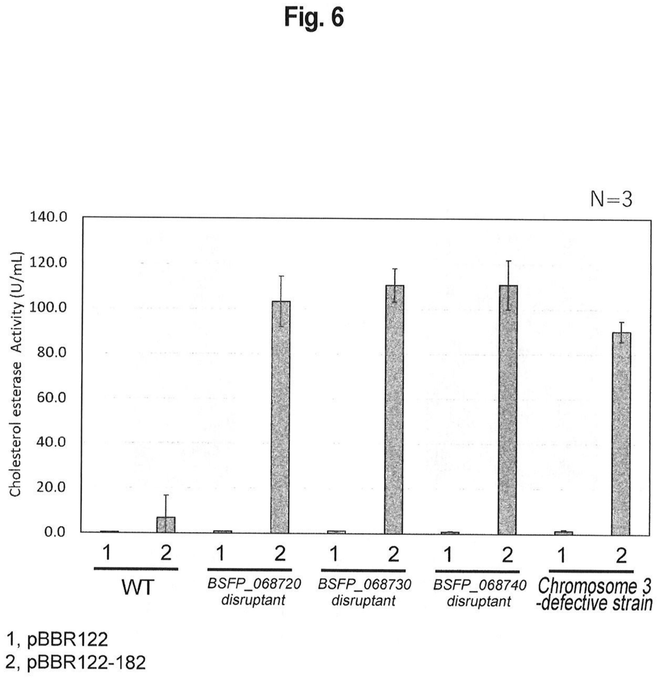

The culture of the bacterial cells of each strain was independently performed three times, and the activity of cholesterol esterase produced by each strain was measured (Figure 6).

As shown in Figure 6, high cholesterol esterase activity and an improved amount of cholesterol esterase produced were found in the BSFP_068720, BSFP_068730, or BSFP_068740 disruptant transfected with the cholesterol esterase expression vector as compared with the wild-type strain transfected with the cholesterol esterase expression vector. High cholesterol esterase activity and an improved amount of cholesterol esterase produced were also obtained in the mutant with chromosome 3 including BSFP_068720, BSFP_068730, and BSFP_068740 being deleted, transfected with the cholesterol esterase expression vector as compared with the wild-type strain transfected with the cholesterol esterase expression vector. This revealed that provided that at least any one of BSFP_068720, BSFP_068730, and BSFP_068740 is disrupted so as to lose its functions, the amount of cholesterol esterase produced is improved even if a gene other than the three genes is disrupted so as to lose its functions. In Examples 5 and 6, the amount of cholesterol esterase produced was presumably improved by increasing the copy number of a plasmid and increasing stability thereof.

### Example 8 Confirmation of effect on other plasmids

In order to confirm an effect of BSFP_068720, BSFP_068730, or BSFP_068740 disruption on a plasmid other than pBBR122, plasmid stability was confirmed using a plasmid pSa (Datta, American society for Microbiology, Washington, DC, pp. 9-15 (1975); Gorai et al., Plasmid 2 489-492 (1979); Ward and Grinsted, Plasmid 7 239-250 (1982); and Tait et al., Mol. Gen. Genet. 186 10-15 (1982)) having an initiation site of replication different from that of pBBR122. A region containing the origin of pSa and the same kanamycin resistance gene and chloramphenicol resistance gene as those of pBBR122 was prepared by genetic total synthesis to obtain pSa plasmid shown in SEQ ID NO: 65.

The respective competent cells of the wild-type strain, the BSFP_068720 disruptant, the BSFP_068730 disruptant, the BSFP_068740 disruptant, and the chromosome 3 deletion mutant were transfected with the pSa plasmid by electroporation in the same manner as in Example 4 to obtain transformants wild-type strain-pSa, BSFP_068720 disruptant-pSa, BSFP_068730 disruptant-pSa, BSFP_068740 disruptant-pSa, and chromosome 3 deletion mutant-pSa.

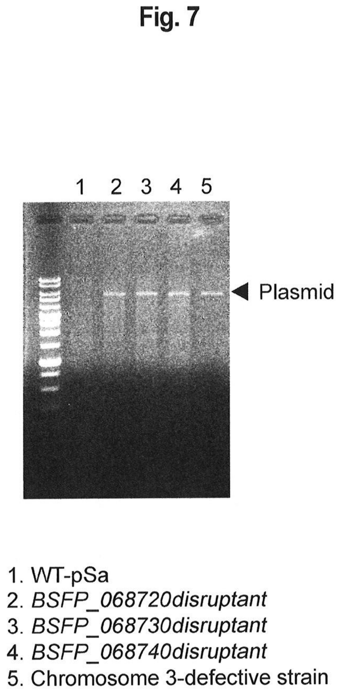

These transformants were each inoculated to 5 mL of LB medium containing 100 µg/mL kanamycin and cultured at 28°C for 12 hours. The resulting bacterial solution was added at a bacterial cell turbidity of OD = 0.2 to 5 mL of LB medium containing 100 µg/mL kanamycin and cultured at 28°C for 24 hours or 48 hours to obtain bacterial cells of each strain differing in culture time. The plasmid was extracted from the bacterial cells cultured for hours using QIAprep Spin Miniprep Kit (manufactured by Qiagen N.V.). The obtained plasmid was cleaved with XhoI (manufactured by Takara Bio Inc.) and subjected to agarose gel electrophoresis (Figure 7). As a result, no signal derived from the plasmid was seen in wild-type strain-pSa, whereas a single signal derived from the plasmid was seen in BSFP_068720 disruptant-pSa, BSFP_068730 disruptant-pSa, BSFP_068740 disruptant-pSa, and chromosome 3 deletion mutant-pSa.

These results indicated that a signal obtained in agarose electrophoresis was stronger in the BSFP_068720, BSFP_068730, or BSFP_068740 disruptant transfected with pSa than in the wild-type strain transfected with pSa. The results also indicated that the copy number of the plasmid was also larger in the mutant with chromosome 3 including BSFP_068720, BSFP_068730, and BSFP_068740 being deleted, transfected with pSa than in the wild-type strain transfected with pSa. This also indicated that functional deletion of at least any one of BSFP_068720, BSFP_068730, and BSFP_068740 increases the plasmid copy number of not only pBBR122 but the pSa plasmid.

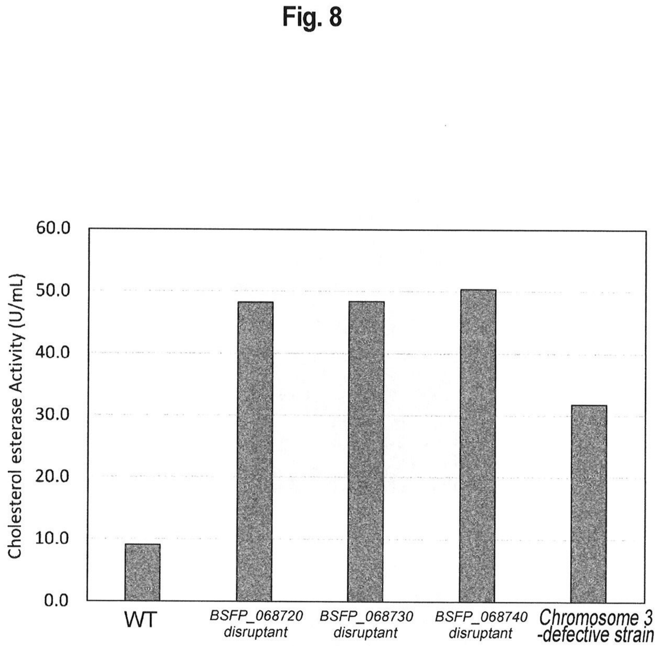

In order to further confirm an effect on another plasmid, cholesterol esterase activity was confirmed using a plasmid having RK2ori different from the initiation site of replication of pBBR122 (T. J. Schmidhauser et al., Plasmid. 9 (3) 325-30 (1983)). Regions necessary for the plasmid having RK2ori, i.e., RK2ori, a region containing trpA, and a region containing the kanamycin resistance gene of pBBR122, 182 promoter, cholesterol esterase gene, foldase gene thereof, and a terminator region were prepared by genetic total synthesis to obtain pRK2-182 plasmid shown in SEQ ID NO: 66. Each disruptant was transformed in the same manner as in Example 4, and cholesterol esterase activity after culture at 28°C for 72 hours was confirmed in the same manner as in Example 7. As shown in Figure 8, high cholesterol esterase activity and an improved amount of cholesterol esterase produced were found in the BSFP_068720, BSFP_068730, or BSFP_068740 disruptant transfected with the cholesterol esterase expression vector as compared with the wild-type strain transfected with the cholesterol esterase expression vector. High cholesterol esterase activity and an improved amount of cholesterol esterase produced were also obtained in the mutant with chromosome 3 including BSFP_068720, BSFP_068730, and BSFP_068740 being deleted, transfected with the cholesterol esterase expression vector as compared with the wild-type strain transfected with the cholesterol esterase expression vector. This revealed that provided that at least any one of BSFP_068720, BSFP_068730, and BSFP_068740 is disrupted so as to lose its functions, the amount of cholesterol esterase produced is improved even if a gene other than the three genes is disrupted so as to lose its functions. This indicated that functional deletion of at least any one of BSFP_068720, BSFP_068730, and BSFP_068740 improves the cholesterol esterase productivity of not only pBBR122 but the pRK2 plasmid.

From these results, similar increase in copy number was seen in the plasmids pSa and pRK2 other than pBBR122, and cholesterol esterase productivity was presumably thereby improved.

### Example 9 Confirmation of effect on expression of other proteins

In order to confirm an effect of BSFP_068720, BSFP_068730, or BSFP_068740 disruption on the expression of a protein other than cholesterol esterase, a DNA sequence containing lipase gene derived from *Burkholderia plantarii* (ATCC43733) shown in SEQ ID NO: 67 and its chaperon gene was totally synthesized. PCR was performed using the totally synthesized gene as a template and primers shown in SEQ ID NOs: 68 and 69, and amplification fragments were excised by agarose gel electrophoresis and purified using QIAquick Gel Extraction Kit (manufactured by Qiagen N.V.) to obtain a BpLip (*Burkholderia plantarii* lipase) fragment. PCR was performed using pBBR122-182 as a template and primers shown in SEQ ID NOs: 70 and 71, and agarose gel electrophoresis and purification using QIAquick Gel Extraction Kit (manufactured by Qiagen N.V.) were performed in the same manner as above. The obtained sequence and the BpLip fragment were linked using InFusion(R) HD Cloning Kit (manufactured by Takara Bio Inc.), and *E*. *coli* DH5α was transformed therewith and then spread over LB agar medium containing 50 µg/mL kanamycin. After culture at 30°C for 24 hours, the obtained colonies were inoculated to LB medium containing 50 µg/mL kanamycin and cultured at 37°C for 12 hours, followed by plasmid extraction from the culture solution using QIAprep Spin Miniprep Kit (manufactured by Qiagen N.V.) to obtain pBBR122-BpLip.

A DNA sequence containing lipase gene derived from *Burkholderia cepacia* shown in SEQ ID NO: 72 was totally synthesized. PCR was performed using the totally synthesized gene as a template and primers shown in SEQ ID NOs: 73 and 74, and amplification fragments were excised by agarose gel electrophoresis and purified using QIAquick Gel Extraction Kit (manufactured by Qiagen N.V.) to obtain a BcLip (*Burkholderia cepacia* lipase) fragment. PCR was performed using pBBR122-182 as a template and primers shown in SEQ ID NOs: 70 and 71, and agarose gel electrophoresis and purification using QIAquick Gel Extraction Kit (manufactured by Qiagen N.V.) were performed in the same manner as above. The obtained sequence and the BcLip fragment were linked using InFusion(R) HD Cloning Kit (manufactured by Takara Bio Inc.), and E. *coli* DH5α was transformed therewith and then spread over LB agar medium containing 50 µg/mL kanamycin. After culture at 30°C for 24 hours, the obtained colonies were inoculated to LB medium containing 50 µg/mL kanamycin and cultured at 37°C for 12 hours, followed by plasmid extraction from the culture solution using QIAprep Spin Miniprep Kit (manufactured by Qiagen N.V.) to obtain pBBR122-BcLip.

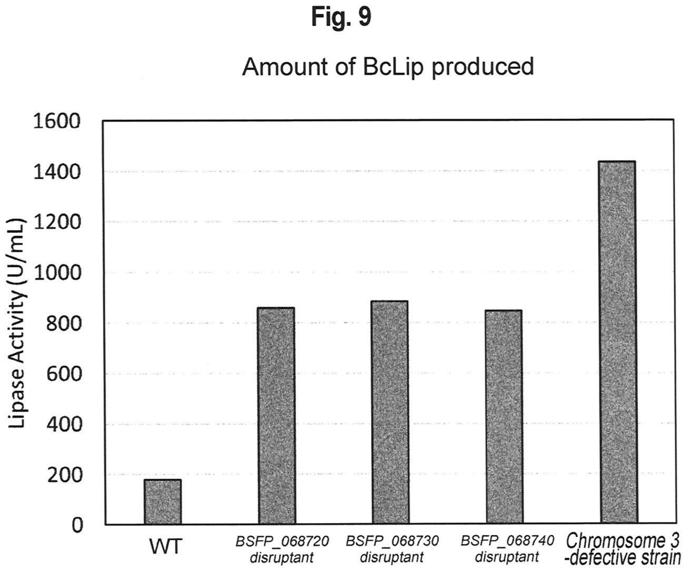

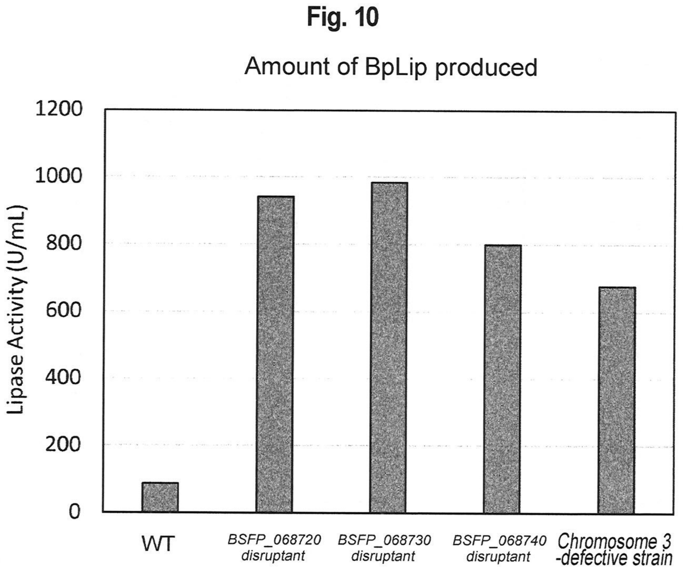

The respective competent cells of the wild-type strain and the chromosome 3 deletion mutant were transfected with pBBR122-BpLip or pBBR122-BcLip by electroporation in the same manner as in Example 4 to obtain transformants wild-type strain-pBBR122-BpLip, chromosome 3 deletion mutant-pBBR122-BpLip, wild-type strain-pBBR122-BcLip, and chromosome 3 deletion mutant-pBBR122-BcLip. The transformants were each inoculated to 5 mL of YS medium (3% yeast extract and 3% sorbitol, pH 7.0) containing 100 µg/mL kanamycin and cultured at 28°C for 72 hours. The amount of lipase produced by each strain thus cultured was evaluated by use of the enzymatic activity measurement method described in T. Yamaguchi et al., Agr. Biol. Chem. 37 (1973) to measure the activity of the lipase produced by each strain (Figures 9 and 10).

As shown in Figures 9 and 10, high lipase activity and an improved amount of lipase produced were obtained in the mutant with chromosome 3 including BSFP_068720, BSFP_068730, and BSFP_068740 being deleted, transfected with the expression vector containing the *Burkholderia* plantarii-derived lipase gene or the *Burkholderia cepacia-derived* lipase gene as compared with the wild-type strain transfected with this expression vector. This revealed that provided that at least any one of BSFP_068720, BSFP_068730, and BSFP_068740 is disrupted so as to lose its functions, the amount of lipase produced is improved even if a gene other than the three genes is disrupted so as to lose its functions. These results indicated that functional deletion of at least any one of BSFP_068720, BSFP_068730, and BSFP_068740 improves the amount of the protein of interest produced from the gene contained in the expression vector.

### Example 10 Preparation of expression vector using novel promoter

DNA amplification was performed by PCR using the *Burkholderia stabilis* total DNA obtained in Example 1 as a template and primers of SEQ ID NOs: 76 and 77 or SEQ ID NOs: 78 and 79 in the sequence listing. As a result, a 14020 promoter DNA fragment and a 48230 promoter DNA fragment, respectively, were obtained as DNA fragments of promoter regions. The obtained DNA fragments were subjected to agarose gel electrophoresis, excised from the gel, and purified using QIAquick Gel Extraction Kit (manufactured by Qiagen N.V.). DNA amplification was performed using pBBR122-182 as a template and primers of SEQ ID NOs: 80 and 81 to amplify a DNA fragment of the expression vector. The obtained expression vector DNA fragment and the 14020 promoter DNA fragment or the expression vector DNA fragment and the 48230 promoter DNA fragment were linked using In-Fusion(R) HD Cloning Kit (manufactured by Takara Bio Inc.). *E. coli* DH5α was transformed with each linked DNA and then spread over LB agar medium containing 50 µg/mL kanamycin. After culture at 30°C for 24 hours, the obtained colonies were inoculated to LB medium containing 50 µg/mL kanamycin and cultured at 37°C for 12 hours, followed by plasmid extraction from the culture solution using QIAprep Spin Miniprep Kit (manufactured by Qiagen N.V.) to obtain expression vectors pBBR122-14020 and pBBR122-48230 in which the promoter upstream of the cholesterol esterase gene was replaced with each novel promoter.

### Example 11 Confirmation of activity of cholesterol esterase produced through novel promoter

The competent cells of the chromosome 3 deletion mutant prepared in Example 4 were thawed on ice, and the pBBR122-14020 or the pBBR122-48230 obtained in Example 10 was added thereto in an amount of approximately 200 to 400 ng and mixed. This mixed solution was transferred to a 0.2 cm wide electroporation cuvette (manufactured by Bio-Rad Laboratories, Inc.) and given electric pulse at an electric field intensity of 12.5 kV/cm, a capacitance of 25 µF, and an external resistance of 200 Ω using a gene transduction apparatus Gene Pulser II. The mixed solution of the bacterial cells and the DNA treated with electric pulse was mixed with 1 mL of LB medium and cultured at 30°C for 1 hour. Then, 200 µL of the bacterial solution was spread over LB medium containing 50 µg/mL kanamycin and cultured at 30°C for 1 day to obtain transformants chromosome 3 deletion mutant-pBBR122-14020 and chromosome 3 deletion mutant-pBBR122-48230.

These transformants and the chromosome 3 deletion mutant-pBBR122-182 obtained in Example 4 were each inoculated to 5 mL of a medium (4% yeast extract, 1.5% sorbitol, 1.5% glycerol, and 5% methyl oleate, pH 6.5) containing 100 µg/mL kanamycin and cultured at 28°C for 72 hours. The amount of cholesterol esterase produced by each strain thus cultured was evaluated by use of the enzymatic activity measurement method described in Example 7.

**[Table 2]**

| | Cholesterol esterase activity (U/mL) |
|---|---|
| Chromosome 3 deletion mutant-pBBR122-182 | 111.0 |
| Chromosome 3 deletion mutant-pBBR122-14020 | 146.4 |
| Chromosome 3 deletion mutant-pBBR122-48230 | 145.2 |

As shown in Table 2, the amount of cholesterol esterase produced was higher in the transformants using the 14020 promoter or the 48230 promoter as a promoter than in the transformant using the cholesterol esterase expression vector pBBR122-182 described in Japanese Patent Laid-Open No. 2019-205402 (supra) as a promoter. This indicated that the 14020 promoter and the 48230 promoter are very useful for the protein expression of interest.

### Example 12 Confirmation of expression level depending on difference in length of novel promoter

Expression vectors having a shortened length of each novel promoter were prepared by the following method. DNA amplification was performed in the same manner as in Example 10 by PCR using the *Burkholderia stabilis* total DNA obtained in Example 1 as a template and a primer set of SEQ ID NOs: 82 and 77, SEQ ID NOs: 83 and 77, SEQ ID NOs: 84 and 77, SEQ ID NOs: 85 and 77, SEQ ID NOs: 86 and 77, SEQ ID NOs: 87 and 77, SEQ ID NOs: 88 and 77, SEQ ID NOs: 89 and 77, SEQ ID NOs: 90 and 77, SEQ ID NOs: 91 and 77, SEQ ID NOs: 92 and 77, SEQ ID NOs: 93 and 77, SEQ ID NOs: 94 and 77, SEQ ID NOs: 95 and 77, SEQ ID NOs: 96 and 77, or SEQ ID NOs: 97 and 77, and agarose electrophoresis and extraction from gel were performed to obtain DNA fragments. These DNA fragments were designated as 14020-250, 14020-200, 14020-150, 14020-100, 14020-90, 14020-80, 14020-70, 14020-60, 14020-50, 48230-250, 48230-200, 48230-150, 48230-140, 48230-130, 48230-120, and 48230-110, respectively. Each fragment is indicated by "promoter name-promoter length". For example, 14020-250 is a DNA fragment for preparing 14020 promoter with a length set to 250 bp.

Each of the DNA fragments 14020-250, 14020-200, 14020-150, 14020-100, 14020-90, 14020-80, 14020-70, 14020-60, 14020-50, 48230-250, 48230-200, 48230-150, 48230-140, 48230-130, 48230-120, and 48230-110 and a DNA fragment of an expression vector obtained by DNA amplification using the pBBR122-182 prepared in Example 10 as a template and primers of SEQ ID NOs: 80 and 81 were linked in the same manner as in Example 10 using InFusion(R) HD Cloning Kit (manufactured by Takara Bio Inc.), and transformation of E. *coli,* culture, and plasmid extraction were performed in the same manner as in Example 10 to obtain expression vectors pBBR122-14020-250, pBBR122-14020-200, pBBR122-14020-150, pBBR122-14020-100, pBBR122-14020-90, pBBR122-14020-80, pBBR122-14020-70, pBBR122-14020-60, pBBR122-14020-50, pBBR122-48230-250, pBBR122-48230-200, pBBR122-48230-150, pBBR122-48230-140, pBBR122-48230-130, pBBR122-48230-120, and pBBR122-48230-110.

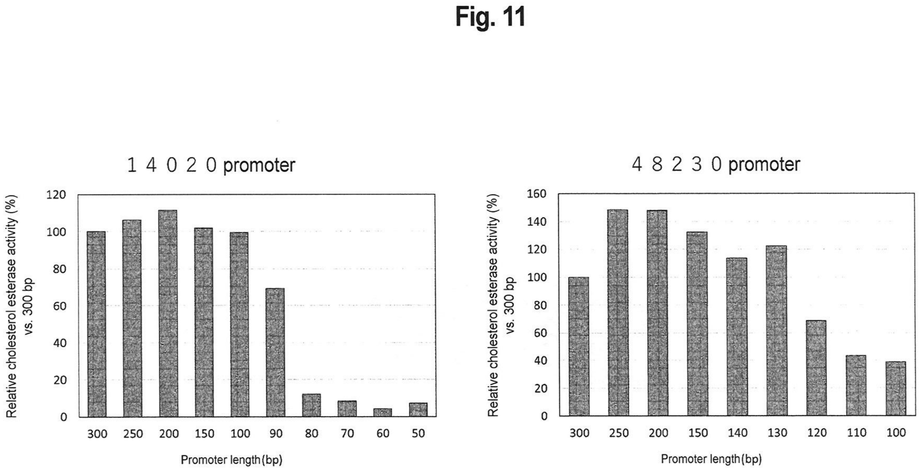

The competent cells of the chromosome 3 deletion mutant were transformed with each of these expression vectors in the same manner as in Example 11 to obtain transformants chromosome 3 deletion mutant-pBBR122-14020-250, chromosome 3 deletion mutant-pBBR122-14020-200, chromosome 3 deletion mutant-pBBR122-14020-150, chromosome 3 deletion mutant-pBBR122-14020-100, chromosome 3 deletion mutant-pBBR122-14020-90, chromosome 3 deletion mutant-pBBR122-14020-80, chromosome 3 deletion mutant-pBBR122-14020-70, chromosome 3 deletion mutant-pBBR122-14020-60, chromosome 3 deletion mutant-pBBR122-14020-50, chromosome 3 deletion mutant-pBBR122-48230-250, chromosome 3 deletion mutant-pBBR122-48230-200, chromosome 3 deletion mutant-pBBR122-48230-150, chromosome 3 deletion mutant-pBBR122-48230-140, chromosome 3 deletion mutant-pBBR122-48230-130, chromosome 3 deletion mutant-pBBR122-48230-120, and chromosome 3 deletion mutant-pBBR122-48230-110 having a shortened length of each promoter. These transformants and the chromosome 3 deletion mutant-pBBR122-14020 and the chromosome 3 deletion mutant-pBBR122-48230 obtained in Example 10 were each cultured by the same method as in Example 11, and the amount of cholesterol esterase produced by each strain was evaluated by use of the enzymatic activity measurement method described in Example 7. Relative cholesterol esterase activity vs. the 300 bp long promoter was determined as to the 14020 promoter according to the following expression (Figure 11). (Activity of cholesterol esterase produced by the transformant) / (Activity of cholesterol esterase produced by chromosome 3 deletion mutant-pBBR122-14020) × 100

Likewise, relative cholesterol esterase activity vs. the 300 bp long promoter was determined as to the 48230 promoter according to the following expression. (Activity of cholesterol esterase produced by the transformant) / (Activity of cholesterol esterase produced by chromosome 3 deletion mutant-pBBR122-48230) × 100

As shown in Figure 11, cholesterol esterase was produced even when the 14020 promoter and the 48230 promoter were shorter than 300 bp.

### Example 13 Confirmation of effect on other bacteria of genus Burkholderia

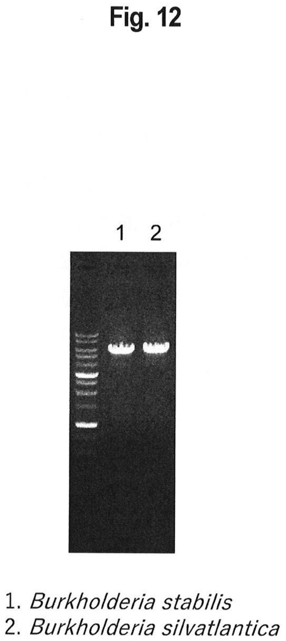

In order to confirm that the present invention is also applicable to other bacteria of the genus *Burkholderia,* whether a bacterium of the genus *Burkholderia* other than *Burkholderia stabilis* retained BSFP_068720, BSFP_068730, and BSFP_068740 was examined. Total DNA was extracted from *Burkholderia silvatlantica* (NBRC106337) in the same manner as in Example 1. DNA amplification was performed by PCR using the obtained *Burkholderia silvatlantica* total DNA or the *Burkholderia stabilis* total DNA obtained in Example 1 as a template and primers of SEQ ID NOs: 98 and 99 capable of amplifying a region including BSFP_068720, BSFP_068730, and BSFP_068740. As a result of subjecting the obtained DNA fragments to agarose gel electrophoresis, DNA amplification was also observed in *Burkholderia silvatlantica,* as in *Burkholderia stabilis* (Figure 12). From this result, *Burkholderia silvatlantica* retains homologous genes of BSFP_068720, BSFP_068730, and BSFP_068740, and the disruption of these genes can be expected to enhance production or increase a copy number of an expression construct.

### Preferred Embodiments

### [Embodiment 1]

A method for producing a protein encoded by a target gene, comprising the step of
expressing the target gene in a bacterium of the genus *Burkholderia,* wherein the bacterium lacks one or more genes selected from the group consisting of BSFP_068740, BSFP_068730, and BSFP_068720, or has inhibited expression of the genes or proteins encoded by the genes.

### [Embodiment 2]

The method according to embodiment 1, wherein
the gene BSFP_068740 comprises any one of the following DNAs (i) to (viii):
(i) DNA consisting of the nucleotide sequence of SEQ ID NO: 1;
(ii) DNA capable of hybridizing under stringent conditions to DNA consisting of a nucleotide sequence complementary to the nucleotide sequence of SEQ ID NO: 1, wherein a protein encoded thereby has a function of decreasing a copy number of an expression construct in the bacterium of the genus *Burkholderia,* or reducing stability thereof;
(iii) DNA consisting of a nucleotide sequence derived from the nucleotide sequence of SEQ ID NO: 1 by the deletion, substitution, or addition of one or a few bases, wherein a protein encoded thereby has a function of decreasing a copy number of an expression construct in the bacterium of the genus *Burkholderia,* or reducing stability thereof;
(iv) DNA consisting of a nucleotide sequence having at least 80% or higher sequence identity to SEQ ID NO: 1, wherein a protein encoded thereby has a function of decreasing a copy number of an expression construct in the bacterium of the genus *Burkholderia,* or reducing stability thereof;
(v) DNA consisting of a nucleotide sequence encoding a protein consisting of the amino acid sequence of SEQ ID NO: 2;
(vi) DNA capable of hybridizing under stringent conditions to DNA consisting of a nucleotide sequence encoding a protein consisting of the amino acid sequence of SEQ ID NO: 2, wherein a protein encoded thereby has a function of decreasing a copy number of an expression construct in the bacterium of the genus *Burkholderia,* or reducing stability thereof;
(vii) DNA consisting of a nucleotide sequence encoding a protein consisting of an amino acid sequence derived from the amino acid sequence of SEQ ID NO: 2 by the deletion, substitution, or addition of one or a few amino acids, wherein the protein has a function of decreasing a copy number of an expression construct in the bacterium of the genus *Burkholderia,* or reducing stability thereof; and (viii) DNA consisting of a nucleotide sequence encoding a protein consisting of an amino acid sequence having at least 80% or higher sequence identity to SEQ ID NO: 2, wherein the protein has a function of decreasing a copy number of an expression construct in the bacterium of the genus *Burkholderia,* or reducing stability thereof.

### [Embodiment 3]

The method according to embodiment 1 or 2, wherein
the gene BSFP_068730 comprises any one of the following DNAs (i) to (viii):
(i) DNA consisting of the nucleotide sequence of SEQ ID NO: 3;
(ii) DNA capable of hybridizing under stringent conditions to DNA consisting of a nucleotide sequence complementary to the nucleotide sequence of SEQ ID NO: 3, wherein a protein encoded thereby has a function of decreasing a copy number of an expression construct in the bacterium of the genus *Burkholderia,* or reducing stability thereof;
(iii) DNA consisting of a nucleotide sequence derived from the nucleotide sequence of SEQ ID NO: 3 by the deletion, substitution, or addition of one or a few bases, wherein a protein encoded thereby has a function of decreasing a copy number of an expression construct in the bacterium of the genus *Burkholderia,* or reducing stability thereof;
(iv) DNA consisting of a nucleotide sequence having at least 80% or higher sequence identity to SEQ ID NO: 3, wherein a protein encoded thereby has a function of decreasing a copy number of an expression construct in the bacterium of the genus *Burkholderia,* or reducing stability thereof;
(v) DNA consisting of a nucleotide sequence encoding a protein consisting of the amino acid sequence of SEQ ID NO: 4;
(vi) DNA capable of hybridizing under stringent conditions to DNA consisting of a nucleotide sequence encoding a protein consisting of the amino acid sequence of SEQ ID NO: 4, wherein a protein encoded thereby has a function of decreasing a copy number of an expression construct in the bacterium of the genus *Burkholderia,* or reducing stability thereof;
(vii) DNA consisting of a nucleotide sequence encoding a protein consisting of an amino acid sequence derived from the amino acid sequence of SEQ ID NO: 4 by the deletion, substitution, or addition of one or a few amino acids, wherein the protein has a function of decreasing a copy number of an expression construct in the bacterium of the genus *Burkholderia,* or reducing stability thereof; and
(viii) DNA consisting of a nucleotide sequence encoding a protein consisting of an amino acid sequence having at least 80% or higher sequence identity to SEQ ID NO: 4, wherein the protein has a function of decreasing a copy number of an expression construct in the bacterium of the genus *Burkholderia,* or reducing stability thereof.

### [Embodiment 4]

The method according to any one of embodiments 1 to 3, wherein
the gene BSFP_068720 comprises any one of the following DNAs (i) to (viii):
(i) DNA consisting of the nucleotide sequence of SEQ ID NO: 5;
(ii) DNA capable of hybridizing under stringent conditions to DNA consisting of a nucleotide sequence complementary to the nucleotide sequence of SEQ ID NO: 5, wherein a protein encoded thereby has a function of decreasing a copy number of an expression construct in the bacterium of the genus *Burkholderia,* or reducing stability thereof;
(iii) DNA consisting of a nucleotide sequence derived from the nucleotide sequence of SEQ ID NO: 5 by the deletion, substitution, or addition of one or a few bases, wherein a protein encoded thereby has a function of decreasing a copy number of an expression construct in the bacterium of the genus *Burkholderia,* or reducing stability thereof;
(iv) DNA consisting of a nucleotide sequence having at least 80% or higher sequence identity to SEQ ID NO: 5, wherein a protein encoded thereby has a function of decreasing a copy number of an expression construct in the bacterium of the genus *Burkholderia,* or reducing stability thereof;
(v) DNA consisting of a nucleotide sequence encoding a protein consisting of the amino acid sequence of SEQ ID NO: 6;
(vi) DNA capable of hybridizing under stringent conditions to DNA consisting of a nucleotide sequence encoding a protein consisting of the amino acid sequence of SEQ ID NO: 6, wherein a protein encoded thereby has a function of decreasing a copy number of an expression construct in the bacterium of the genus *Burkholderia,* or reducing stability thereof;
(vii) DNA consisting of a nucleotide sequence encoding a protein consisting of an amino acid sequence derived from the amino acid sequence of SEQ ID NO: 6 by the deletion, substitution, or addition of one or a few amino acids, wherein the protein has a function of decreasing a copy number of an expression construct in the bacterium of the genus *Burkholderia,* or reducing stability thereof; and
(viii) DNA consisting of a nucleotide sequence encoding a protein consisting of an amino acid sequence having at least 80% or higher sequence identity to SEQ ID NO: 6, wherein the protein has a function of decreasing a copy number of an expression construct in the bacterium of the genus *Burkholderia,* or reducing stability thereof.

### [Embodiment 5]

The method according to any one of embodiments 1 to 4, wherein the target gene is expressibly linked to the expression construct.

### [Embodiment 6]

The method according to any one of embodiments 2 to 5, wherein the expression construct is an expression vector.

### [Embodiment 7]

The method according to embodiment 6, wherein the expression construct is a plasmid capable of replicating in the bacterium of the genus *Burkholderia.*

### [Embodiment 8]

The method according to embodiment 7, wherein the plasmid is a broad-host-range vector.

### [Embodiment 9]

The method according to embodiment 8, wherein the broad-host-range vector is pBBR122, pSa, or RK2.

### [Embodiment 10]

The method according to any one of embodiments 2 to 9, wherein the expression construct comprises a promoter that controls the expression of the target gene.

### [Embodiment 11]

The method according to embodiment 10, wherein the promoter is derived from the bacterium of the genus *Burkholderia.*

### [Embodiment 12]

The method according to any one of embodiments 1 to 11, wherein
the promoter comprises any one of the following DNAs (i) to (iv):
(i) DNA that consists of a sequence of more than 80 consecutive nucleotides in the nucleotide sequence of SEQ ID NO: 7, and has promoter activity;
(ii) DNA that is capable of hybridizing under stringent conditions to DNA consisting of a complementary sequence of more than 80 consecutive nucleotides in the nucleotide sequence of SEQ ID NO: 7, and has promoter activity;
(iii) DNA that consists of a nucleotide sequence derived from a sequence of more than 80 consecutive nucleotides in the nucleotide sequence of SEQ ID NO: 7 by the deletion, substitution, or addition of one or a few bases, and has promoter activity; and
(iv) DNA that consists of a nucleotide sequence having at least 80% or higher sequence identity to a sequence of more than 80 consecutive nucleotides in SEQ ID NO: 7, and has promoter activity.

### [Embodiment 13]

The method according to embodiment 12, wherein
the promoter comprises any one of the following DNAs (i) to (iv):
(i) DNA consisting of the nucleotide sequence of SEQ ID NO: 8;
(ii) DNA that is capable of hybridizing under stringent conditions to DNA consisting of a nucleotide sequence complementary to the nucleotide sequence of SEQ ID NO: 8, and has promoter activity;
(iii) DNA that consists of a nucleotide sequence derived from the nucleotide sequence of SEQ ID NO: 8 by the deletion, substitution, or addition of one or a few bases, and has promoter activity; and
(iv) DNA that consists of a nucleotide sequence having at least 80% or higher sequence identity to SEQ ID NO: 8, and has promoter activity.

### [Embodiment 14]

The method according to any one of embodiments 1 to 13, wherein
the promoter comprises any one of the following DNAs (i) to (iv):
(i) DNA that consists of a sequence of more than 100 consecutive nucleotides in the nucleotide sequence of SEQ ID NO: 9, and has promoter activity;
(ii) DNA that is capable of hybridizing under stringent conditions to DNA consisting of a complementary sequence of more than 100 consecutive nucleotides in the nucleotide sequence of SEQ ID NO: 9, and has promoter activity;
(iii) DNA that consists of a nucleotide sequence derived from a sequence of more than 100 consecutive nucleotides in the nucleotide sequence of SEQ ID NO: 9 by the deletion, substitution, or addition of one or a few bases, and has promoter activity; and
(iv) DNA that consists of a nucleotide sequence having at least 80% or higher sequence identity to a sequence of more than 100 consecutive nucleotides in SEQ ID NO: 9, and has promoter activity.

### [Embodiment 15]

The method according to embodiment 14, wherein
the promoter comprises any one of the following DNAs (i) to (iv):
(i) DNA consisting of the nucleotide sequence of SEQ ID NO: 10;
(ii) DNA that is capable of hybridizing under stringent conditions to DNA consisting of a nucleotide sequence complementary to the nucleotide sequence of SEQ ID NO: 10, and has promoter activity;
(iii) DNA that consists of a nucleotide sequence derived from the nucleotide sequence of SEQ ID NO: 10 by the deletion, substitution, or addition of one or a few bases, and has promoter activity; and
(iv) DNA that consists of a nucleotide sequence having at least 80% or higher sequence identity to SEQ ID NO: 10, and has promoter activity.

### [Embodiment 16]

The method according to any one of embodiments 1 to 15, wherein the target gene encodes esterase.

### [Embodiment 17]

The method according to embodiment 16, wherein
the gene encoding esterase comprises any one of the following DNAs (i) to (iv):
(i) DNA consisting of a nucleotide sequence of any one of SEQ ID NOs: 11 to 13;
(ii) DNA capable of hybridizing under stringent conditions to DNA consisting of a nucleotide sequence complementary to a nucleotide sequence of any one of SEQ ID NOs: 11 to 13, wherein a protein encoded thereby has esterase activity;
(iii) DNA consisting of a nucleotide sequence derived from a nucleotide sequence of any one of SEQ ID NOs: 11 to 13 by the deletion, substitution, or addition of one or a few bases, wherein a protein encoded thereby has esterase activity; and
(iv) DNA consisting of a nucleotide sequence having at least 80% or higher sequence identity to a nucleotide sequence of any one of SEQ ID NOs: 11 to 13, wherein a protein encoded thereby has esterase activity.

### [Embodiment 18]

The method according to any one of embodiments 1 to 17, wherein the expression construct comprises DNA encoding foldase.

### [Embodiment 19]

The method according to embodiment 18, wherein
the DNA encoding foldase comprises any one of the following DNAs (i) to (iv):
(i) DNA consisting of a nucleotide sequence of any one of SEQ ID NOs: 14 to 16;
(ii) DNA capable of hybridizing under stringent conditions to DNA consisting of a nucleotide sequence complementary to a nucleotide sequence of any one of SEQ ID NOs: 14 to 16, wherein a protein encoded thereby has foldase activity;
(iii) DNA consisting of a nucleotide sequence derived from a nucleotide sequence of any one of SEQ ID NOs: 14 to 16 by the deletion, substitution, or addition of one or a few bases, wherein a protein encoded thereby has foldase activity; and
(iv) DNA consisting of a nucleotide sequence having at least 80% or higher sequence identity to a nucleotide sequence of any one of SEQ ID NOs: 14 to 16, wherein a protein encoded thereby has foldase activity.

### [Embodiment 20]

The method according to any one of embodiments 1 to 19, wherein the target gene further encodes a signal sequence.

### [Embodiment 21]

The method according to any one of embodiments 1 to 20, wherein the bacterium of the genus *Burkholderia* is *Burkholderia stabilis.*

### [Embodiment 22]

A promoter consisting of any one of the following DNAs (i) to (iv):
(i) DNA that consists of a sequence of more than 80 consecutive nucleotides in the nucleotide sequence of SEQ ID NO: 7, and has promoter activity;
(ii) DNA that is capable of hybridizing under stringent conditions to DNA consisting of a complementary sequence of more than 80 consecutive nucleotides in the nucleotide sequence of SEQ ID NO: 7, and has promoter activity;
(iii) DNA that consists of a nucleotide sequence derived from a sequence of more than 80 consecutive nucleotides in the nucleotide sequence of SEQ ID NO: 7 by the deletion, substitution, or addition of one or a few bases, and has promoter activity; and
(iv) DNA that consists of a nucleotide sequence having at least 80% or higher sequence identity to a sequence of more than 80 consecutive nucleotides in SEQ ID NO: 7, and has promoter activity.

### [Embodiment 23]

The promoter according to embodiment 22, wherein
the promoter comprises any one of the following DNAs (i) to (iv):
(i) DNA consisting of the nucleotide sequence of SEQ ID NO: 8;
(ii) DNA that is capable of hybridizing under stringent conditions to DNA consisting of a nucleotide sequence complementary to the nucleotide sequence of SEQ ID NO: 8, and has promoter activity;
(iii) DNA that consists of a nucleotide sequence derived from the nucleotide sequence of SEQ ID NO: 8 by the deletion, substitution, or addition of one or a few bases, and has promoter activity; and
(iv) DNA that consists of a nucleotide sequence having at least 80% or higher sequence identity to SEQ ID NO: 8, and has promoter activity.

### [Embodiment 24]

A promoter consisting of any one of the following DNAs (i) to (iv):
(i) DNA that consists of a sequence of more than 100 consecutive nucleotides in the nucleotide sequence of SEQ ID NO: 9, and has promoter activity;
(ii) DNA that is capable of hybridizing under stringent conditions to DNA consisting of a complementary sequence of more than 100 consecutive nucleotides in the nucleotide sequence of SEQ ID NO: 9, and has promoter activity;
(iii) DNA that consists of a nucleotide sequence derived from a sequence of more than 100 consecutive nucleotides in the nucleotide sequence of SEQ ID NO: 9 by the deletion, substitution, or addition of one or a few bases, and has promoter activity; and
(iv) DNA that consists of a nucleotide sequence having at least 80% or higher sequence identity to a sequence of more than 100 consecutive nucleotides in SEQ ID NO: 9, and has promoter activity.

### [Embodiment 25]

The promoter according to embodiment 24, wherein
the promoter comprises any one of the following DNAs (i) to (iv):
(i) DNA consisting of the nucleotide sequence of SEQ ID NO: 10;
(ii) DNA that is capable of hybridizing under stringent conditions to DNA consisting of a nucleotide sequence complementary to the nucleotide sequence of SEQ ID NO: 10, and has promoter activity;
(iii) DNA that consists of a nucleotide sequence derived from the nucleotide sequence of SEQ ID NO: 10 by the deletion, substitution, or addition of one or a few bases, and has promoter activity; and
(iv) DNA that consists of a nucleotide sequence having at least 80% or higher sequence identity to SEQ ID NO: 10, and has promoter activity.

### [Embodiment 26]

A mutant strain of a bacterium of the genus *Burkholderia* engineered to have inhibited expression of one or more genes selected from the group consisting of BSFP_068740, BSFP_068730, and BSFP_068720 or proteins encoded by the genes.

### [Embodiment 27]

The mutant strain according to embodiment 26, wherein the mutant strain comprises an expression construct, and the expression construct comprises a promoter that controls the expression of the target gene.

### [Embodiment 28]

The mutant strain according to embodiment 27, wherein the promoter is a promoter according to any one of embodiments 22 to 25.

### [Embodiment 29]

A method for maintaining an expression construct in a bacterium of the genus *Burkholderia,* comprising the step of
culturing a bacterium of the genus *Burkholderia,* wherein the bacterium of the genus *Burkholderia* lacks one or more genes selected from the group consisting of BSFP_068740, BSFP_068730, and BSFP_068720, or has inhibited expression of the genes or proteins encoded by the genes, and the bacterium comprises the expression construct.

### [Embodiment 30]

The method according to embodiment 29, wherein the cultured bacterium of the genus *Burkholderia* has an increased copy number of the expression construct or increased stability thereof.

## Claims

1. A promoter consisting of any one of the following DNAs (i) to (iv):
(i) DNA that consists of a sequence of more than 100 consecutive nucleotides in the nucleotide sequence of SEQ ID NO: 9, and has promoter activity;
(ii) DNA that is capable of hybridizing under stringent conditions to DNA consisting of a complementary sequence of more than 100 consecutive nucleotides in the nucleotide sequence of SEQ ID NO: 9, and has promoter activity;
(iii) DNA that consists of a nucleotide sequence derived from a sequence of more than 100 consecutive nucleotides in the nucleotide sequence of SEQ ID NO: 9 by the deletion, substitution, or addition of one or a few bases, and has promoter activity; and
(iv) DNA that consists of a nucleotide sequence having at least 80% or higher sequence identity to a sequence of more than 100 consecutive nucleotides in SEQ ID NO: 9, and has promoter activity.

2. The promoter according to claim 1, wherein
the promoter comprises any one of the following DNAs (i) to (iv):
(i) DNA consisting of the nucleotide sequence of SEQ ID NO: 10;
(ii) DNA that is capable of hybridizing under stringent conditions to DNA consisting of a nucleotide sequence complementary to the nucleotide sequence of SEQ ID NO: 10, and has promoter activity;
(iii) DNA that consists of a nucleotide sequence derived from the nucleotide sequence of SEQ ID NO: 10 by the deletion, substitution, or addition of one or a few bases, and has promoter activity; and
(iv) DNA that consists of a nucleotide sequence having at least 80% or higher sequence identity to SEQ ID NO: 10, and has promoter activity.
